(19) Europäisches Patentamt
European Patent Office
Office européen des brevets

(11) EP 1 347 054 A1

(12) EUROPEAN PATENT APPLICATION

(43) Date of publication:
**24.09.2003 Bulletin 2003/39**

(21) Application number: **02006086.9**

(22) Date of filing: **18.03.2002**

(51) Int Cl.7: **C12N 15/12**, C12N 15/57, C12N 15/62, C12N 5/10, C12N 1/19, C12N 1/21, C12N 9/64, C07K 14/47, G01N 33/50, G01N 33/574, C12Q 1/68, A61K 38/17, A61K 38/48, A61K 48/00, A61K 31/7088, A61P 35/00

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Deutsches Krebsforschungszentrum Stiftung des öffentlichen Rechts**
**69120 Heidelberg (DE)**

(72) Inventors:
- **Angel, Peter**
  **69181 Leimen (DE)**
- **Hess, Jochen**
  **74909 Meckesheim (DE)**
- **Breitenbach, Ute**
  **20251 Hamburg (DE)**
- **Tuckermann, Jan**
  **68159 Mannheim (DE)**
- **Richter, Hartmut**
  **69151 Neckargemünd (DE)**
- **Fürstenberger, Gerhard**
  **69214 Eppelheim (DE)**

(74) Representative: **Schüssler, Andrea, Dr.**
**Kanzlei Huber & Schüssler**
**Truderinger Strasse 246**
**81825 München (DE)**

Remarks:
The sequence listing, which is published as annex to the application documents, was filed after the date of filing. The applicant has declared that it does not include matter which goes beyond the content of the application as filed.

(54) **TAP-70, a novel marker for epithelial tumors**

(57) Described are a novel marker protein associated with epithelial tumors, TAP-70, belonging to the family of aspartyl proteinases and nucleic acid molecules encoding TAP-70 polypeptides. Moreover, diagnostic and therapeutic uses based on the finding that TAP-70 is overexpressed throughout skin carcinogenesis are described.

EP 1 347 054 A1

SEQUENCE LISTING

```
<110> Deutsches Krebsforschungszentrum

<120> TAP-70, a novel marker for epithelial tumors

<130> K 3033EP

<140> EP 02006086.9
<141> 2002-03-18

<160> 17

<170> PatentIn Ver. 2.1


<210> 1
<211> 343
<212> PRT
<213> Homo sapiens

<400> 1
Met Gly Ser Pro Gly Ala Ser Leu Gly Ile Lys Lys Ala Leu Gln Ser
  1               5                  10                  15

Glu Gln Ala Thr Ala Leu Pro Ala Ser Ala Pro Ala Val Ser Gln Pro
             20                  25                  30

Thr Ala Pro Ala Pro Ser Cys Leu Pro Lys Ala Gly Gln Val Ile Pro
         35                  40                  45

Thr Leu Leu Arg Glu Ala Pro Phe Ser Ser Val Ile Ala Pro Thr Leu
     50                  55                  60

Leu Cys Gly Phe Leu Phe Leu Ala Trp Val Ala Ala Glu Val Pro Glu
 65                  70                  75                  80

Glu Ser Ser Arg Met Ala Gly Ser Gly Ala Arg Ser Glu Glu Gly Arg
                 85                  90                  95

Arg Gln His Ala Phe Val Pro Glu Pro Phe Asp Gly Ala Asn Val Val
            100                 105                 110

Pro Asn Leu Trp Leu His Ser Phe Glu Val Ile Asn Asp Leu Asn His
        115                 120                 125

Trp Asp His Ile Thr Lys Leu Arg Phe Leu Lys Glu Ser Leu Arg Gly
    130                 135                 140

Glu Ala Leu Gly Val Tyr Asn Arg Leu Ser Pro Gln Asp Gln Gly Asp
145                 150                 155                 160

Tyr Gly Thr Val Lys Glu Ala Leu Leu Lys Ala Phe Gly Val Pro Gly
                165                 170                 175

Ala Ala Pro Ser His Leu Pro Lys Glu Ile Val Phe Ala Asn Ser Met
            180                 185                 190

Gly Lys Gly Tyr Tyr Leu Lys Gly Lys Ile Gly Lys Val Pro Val Arg
        195                 200                 205

Phe Leu Val Asp Ser Gly Ala Gln Val Ser Val Val His Pro Asn Leu
    210                 215                 220

Trp Glu Glu Val Thr Asp Gly Asp Leu Asp Thr Leu Gln Pro Phe Glu
225                 230                 235                 240
```

Asn Val Val Lys Val Ala Asn Gly Ala Glu Met Lys Ile Leu Gly Val
245 250 255

Trp Asp Thr Ala Val Ser Leu Gly Lys Leu Lys Leu Lys Ala Gln Phe
260 265 270

Leu Val Ala Asn Ala Ser Ala Glu Glu Ala Ile Ile Gly Thr Asp Val
275 280 285

Leu Gln Asp His Asn Ala Ile Leu Asp Phe Glu His Arg Thr Cys Thr
290 295 300

Leu Lys Gly Lys Lys Phe Arg Leu Leu Pro Val Gly Gly Ser Leu Glu
305 310 315 320

Asp Glu Phe Asp Leu Glu Leu Ile Glu Glu Asp Pro Ser Ser Glu Glu
325 330 335

Gly Arg Gln Glu Leu Ser His
340

<210> 2
<211> 1695
<212> DNA
<213> Homo sapiens

<400> 2
caaggatgga tgcagagggt gagcacccat cctgctagtc cggccggatg ctggcaggag 60
ggcggggtga ggaggggcgg agcttccaga acaaaggaga atggggagcc caggggccag 120
cctaggcatc aaaaaggctc tgcagagtga acaggccaca gcactgcctg cctctgcccc 180
agcagtcagc cagccgaccg cgcctgctcc ctcctgcttg cccaaggccg ggcaagtcat 240
ccccactctg cttcgagagg ccccgttttc cagcgtgatt gcgccgacac tgctctgtgg 300
gtttctcttc ttggcgtggg ttgctgctga ggttccagag gagagcagca ggatggccgg 360
gagcggagcc aggagtgagg aaggccgccg gcagcatgcc ttcgtcccgg aacctttga 420
tggggccaat gtcgtcccaa acctctggct gcacagcttt gaagtcatca atgacctcaa 480
ccattgggac catatcacca agctaaggtt cctgaaagag tccctcagag gagaggccct 540
gggtgtctac aataggctca gtccccagga ccagggagac tatgggactg tgaaagaggc 600
cctcctgaag gcctttgggg tccctggggc tgcccccagc cacctgccca aagagatcgt 660
ctttgccaac agcatgggta agggctacta tctcaagggg aagattggca aagtgcccgt 720
gaggttcctg gtggactctg ggcccaggt ctctgtggtc cacccaaact tgtgggagga 780
ggtcactgat ggcgatctgg acaccctgca gccctttgag aatgtggtaa aggtggccaa 840
tggtgctgaa atgaagatcc tgggtgtctg ggatacagcg gtgtccctag gcaagctgaa 900
gctgaaggca cagttcctag tggccaatgc gagtgccgag gaagccatca ttggcactga 960
tgtgctccag gaccacaatg ctatcctgga ctttgagcac cgcacatgca ccctgaaagg 1020
gaagaagttt cgccttctgc ctgtgggagg gtccctggaa gatgagtttg acctggagct 1080
catagaggag gacccctcct cagaagaagg gcggcaggag ctatcccact gagaagccac 1140
cttttcttta acctcctaaa tattggtggg aagacccacc gctgtggggg gggttgcata 1200
tcctcatggg ggtcactggg cttggccagt ctgcttatca actcttgctc ttctctcccc 1260
tttgcctccc tctgcagggg ccttaatctg cccctggtag gggaggcttc cactgaacag 1320
gcacaggtga gggagagcag gctggcttag agggacaggg tccccatggt catcaagctg 1380
ctgttgatga caaagactca aaggctggaa gagctcccaa ggaagctaga aatgcttgtc 1440
tttgaaagaa ctgtgggacc ccttcagatt ccctgaggta tggcttggtc actctcaggt 1500
cctcaaagcc tgtcttagtt gggctgggtc ctagctgcag ggtctttgtg agggtcacag 1560
ttgctctggg acacctccct gaagagcctt tccacctgta caatcgtatt ttctttctgt 1620
catttgcttt gaagcccatt gtgccttatg ccaataattc aattgctgca aacaccaata 1680
aagattgatt catgg 1695

<210> 3
<211> 409
<212> PRT
<213> Mus musculus

<400> 3
Met Ser Ser His Leu Tyr Pro His Leu Gly Tyr Ser Arg Ala Arg Leu
1 5 10 15

```
Gly Arg Val Pro Arg Leu His Pro Leu Thr Arg Ala Val Leu Leu Thr
            20                  25                  30

Gly Trp Ala Gly Arg Cys Pro Val Gly Thr Glu Gly Glu Ala Pro Ile
        35                  40                  45

Leu Leu Val Arg Gln Asp Ala Gly Arg Arg Ala Gly Leu Gly Val Glu
    50                  55                  60

Leu Leu Glu Gln Arg Arg Met Arg Asn Pro Gly Gly Pro Gly Trp Ala
65                  70                  75                  80

Ser Lys Arg Pro Leu Gln Lys Lys Gln Asn Thr Ala Cys Leu Cys Ala
                85                  90                  95

Gln Gln Pro Ala Arg His Phe Val Pro Ala Pro Phe Asn Ser Ser Arg
            100                 105                 110

Gln Gly Lys Asn Thr Ala Gln Pro Thr Glu Pro Ser Leu Ser Ser Val
        115                 120                 125

Ile Ala Pro Thr Leu Phe Cys Ala Phe Leu Tyr Leu Ala Cys Val Thr
    130                 135                 140

Ala Glu Leu Pro Glu Val Ser Arg Arg Met Ala Thr Ser Gly Val Arg
145                 150                 155                 160

Ser Lys Glu Gly Arg Arg Glu His Ala Phe Val Pro Glu Pro Phe Thr
                165                 170                 175

Gly Thr Asn Leu Ala Pro Ser Leu Trp Leu His Arg Phe Glu Val Ile
            180                 185                 190

Asp Asp Leu Asn His Trp Asp His Ala Thr Lys Leu Arg Phe Leu Lys
        195                 200                 205

Glu Ser Leu Lys Gly Asp Ala Leu Asp Val Tyr Asn Gly Leu Ser Ser
    210                 215                 220

Gln Ala Gln Gly Asp Phe Ser Phe Val Lys Gln Ala Leu Leu Arg Ala
225                 230                 235                 240

Phe Gly Ala Pro Gly Glu Ala Phe Ser Glu Pro Glu Glu Ile Leu Phe
                245                 250                 255

Ala Asn Ser Met Gly Lys Gly Tyr Tyr Leu Lys Gly Lys Val Gly His
            260                 265                 270

Val Pro Val Arg Phe Leu Val Asp Ser Gly Ala Gln Val Ser Val Val
        275                 280                 285

His Pro Ala Leu Trp Glu Glu Val Thr Asp Gly Asp Leu Asp Thr Leu
    290                 295                 300

Arg Pro Phe Asn Asn Val Val Lys Val Ala Asn Gly Ala Glu Met Lys
305                 310                 315                 320

Ile Leu Gly Val Trp Asp Thr Glu Ile Ser Leu Gly Lys Thr Lys Leu
                325                 330                 335

Lys Ala Glu Phe Leu Val Ala Asn Ala Ser Ala Glu Glu Ala Ile Ile
            340                 345                 350

Gly Thr Asp Val Leu Gln Asp His Asn Ala Val Leu Asp Phe Glu His
        355                 360                 365

Arg Thr Cys Thr Leu Lys Gly Lys Lys Phe Arg Leu Leu Pro Val Gly
    370                 375                 380
```

Ser Ser Leu Glu Asp Glu Phe Asp Leu Glu Leu Ile Glu Glu Glu Glu
385 390 395 400

Gly Ser Ser Ala Pro Glu Gly Ser His
405

<210> 4
<211> 1948
<212> DNA
<213> Mus msuculus

<400> 4
tccgcagtta atttagaatg tatgagtcac cttatcaagg caggctgtga gagatgagtg 60
actgcagatg ccttctcttt gccccaagca gtgatggggt gaggccaaag ggtcccctc 120
ttctgaaaca ggtagagacc tgctttctgt ctcctcttct ctaatgataa acatctgaat 180
gtcatcacac ctgtatcctc atctgggcta ttctagggct aggctgggga gggtcccgag 240
gctccatccc ctgacccggg ctgtcttact cactgggtgg gctggcaggt gtcccgtagg 300
tactgagggt gaggcaccaa tcctgctagt caggcaagat gctggcagga gggcggggct 360
aggggtggag cttctagaac aaaggagaat gaggaaccct gggggcccag gttgggcatc 420
aaaaaggccc ctgcagaaga agcagaacac agcctgcctc tgtgcccagc agccagccag 480
acactttgta ccggctccct tcaactcgtc caggcagggc aagaacacgg cccagccgac 540
agagccctcg ctctccagcg tgattgcgcc cacactcttc tgtgcgtttc tttacttggc 600
ttgtgttact gctgaacttc cagaggtgag cagaaggatg gccaccagcg gagtcagaag 660
caaggaagga cgccgggagc atgccttcgt cccagaacct ttcactggta ctaacttagc 720
tcccagcctt tggctgcacc gctttgaagt cattgatgac ctcaaccatt gggatcatgc 780
caccaaactg aggttcctga aagagtcgct caagggagat gccctggatg tctacaatgg 840
actcagttcc caggcccagg gcgatttcag ttttgtgaag caagccctcc tgagggcctt 900
tggggcccct ggggaggcct tcagtgagcc cgaagagatt ttgtttgcca acagcatggg 960
taagggctac taccttaaag ggaaggttgg ccatgtgcct gtgagattcc tggtggactc 1020
tggggctcag gtgtctgtgg ttcaccccgc cttatgggag gaggtcactg atggtgacct 1080
ggatactctt cgtcctttta acaatgtggt caaagtggcc aatggggcag agatgaagat 1140
cttgggtgtg tgggacacag aaattagcct gggcaagaca aagctgaagg ccgagtttct 1200
ggtggccaac gccagcgcag aagaggctat tattggcaca gacgtcttgc aggaccacaa 1260
tgccgtgctg gacttcgaac accgcacctg caccctgaag gggaagaagt tccgcctgct 1320
ccctgtcggg agctccttgg aggatgagtt tgacctggag cttattgagg aagaggaggg 1380
gtcttctgca ccggagggct cccactaaga aaccccattt cttgttccca gcattggtag 1440
ggggactttg tgttgggggg agcagatgtc ctggggggta tcatccggcc tagccagtct 1500
ttacaccggt tctcagtttc cctccttcta caggggcctt gctttgcctt tgtttgggga 1560
gggaggccag cttggtggcc taaagcagtg tccccaaggt ctgcaaagac ttccaaggct 1620
ggcaggagct tctgaggaag ccaggaatgt caatcttgag agaggaccct tttagatccc 1680
ctgaagtatg gctcagtcac tttcacgtcc ccaagcctgc tgagctgagc ctggtcttgg 1740
ctaagaccct cacaatccag atgcttggag gagactggca gctgctctgg gagtcctccc 1800
tgagtcctcc cacctgcaca aggatgctcc ctcctgtcct gtcacttgcc ttgaatctca 1860
tggagcctgt atcaataatt caattatttc aaaacaccaa taaagatctg ttcatgtaaa 1920
aaaaaaaaaa aaaaaaaaaa aaaaaaaa 1948

<210> 5
<211> 45
<212> DNA
<213> Artificial Sequence

<220>
<223> Description of Artificial Sequence: Primer

<400> 5
ttttgtacaa gctttttttt tttttttttt tttttttttt tttnn 45

<210> 6
<211> 44
<212> DNA
<213> Artificial Sequence

<220>
<223> Description of Artificial Sequence: Adaptor

<400> 6

ctaatacgac tcactatagg gctcgagcgg ccgcccgggc aggt 44

<210> 7
<211> 10
<212> DNA
<213> Artificial Sequence

<220>
<223> Description of Artificial Sequence: Adaptor

<400> 7
ggcccgtcca 10

<210> 8
<211> 22
<212> DNA
<213> Artificial Sequence

<220>
<223> Description of Artificial Sequence: Primer

<400> 8
ctaatacgac tcactatagg gc 22

<210> 9
<211> 24
<212> DNA
<213> Artificial Sequence

<220>
<223> Description of Artificial Sequence: Primer

<400> 9
tccgaagcgg ccgcccgggc aggt 24

<210> 10
<211> 42
<212> DNA
<213> Artificial Sequence

<220>
<223> Description of Artificial Sequence: Adaptor

<400> 10
ctaatacgac tcactatagg gcagcgtggt cgcggccgag gt 42

<210> 11
<211> 10
<212> DNA
<213> Artificial Sequence

<220>
<223> Description of Artificial Sequence: Adaptor

<400> 11
gccggctcca 10

<210> 12
<211> 20
<212> DNA
<213> Artificial Sequence

<220>

<223> Description of Artificial Sequence: Primer

<400> 12
agcgtggtcg cggccgaggt 20

<210> 13
<211> 19
<212> DNA
<213> Artificial Sequence

<220>
<223> Description of Artificial Sequence: Primer

<400> 13
gtctttgcca acagcatgg 19

<210> 14
<211> 20
<212> DNA
<213> Artificial Sequence

<220>
<223> Description of Artificial Sequence: Primer

<400> 14
cagagtccac caggaacctc 20

<210> 15
<211> 12
<212> PRT
<213> Artificial Sequence

<220>
<223> Description of Artificial Sequence: Active site of TAP-70 (Mouse/Human)

<400> 15
Phe Leu Val Asp Ser Gly Ala Gln Val Ser Val Val
1 5 10

<210> 16
<211> 19
<212> PRT
<213> Mus musculus

<400> 16
His Val Pro Val Arg Phe Leu Val Asp Ser Gly Ala Gln Val Ser Val
1 5 10 15

Val His Pro

<210> 17
<211> 19
<212> PRT
<213> Homo sapiens

<400> 17
Lys Val Pro Val Arg Phe Leu Val Asp Ser Gly Ala Gln Val Ser Val
1 5 10 15

Val His Pro

## Description

The present invention relates to gene expression in normal cells and cells of epithelial tumors and particularly to a novel marker protein associated with epithelial tumors, TAP-70, belonging to the family of aspartyl proteinases as well as nucleic acid molecules encoding TAP-70. Furthermore, the present invention relates to various diagnostic and therapeutic uses based on the finding that TAP-70 is overexpressed throughout epithelial carcinogenesis.

Cancer is a worldwide problem of enormous impact. For example each year more than 340.000 persons in Germany develop cancer and more than 210.000 die from their disease. By that, cancer still ranks second to diseases of the circulatory system as a cause of male and female deaths. At present, approximately every third death is due to circulatory disease and every fourth death is due to cancer. Epithelial tumors represent the majority of cancer: Lung cancer is the leading cause of cancer deaths in males, and breast cancer is the leading cause in females. The second leading cause of cancer deaths for both sexes is colorectal cancer (Becker, N. and Wahrendorf, J., (1997) Atlas of Cancer Mortality in the Federal Republic of Germany 1981-1990, Springer-Verlag, Berlin, Heidelberg). Further epithelial cancers with leading death rates are prostate, ovarian and pancreas carcinomas.

In most cases diagnosis and monitoring of cancer is difficult because of the heterogeneity of the disease. For diagnosis different grades of malignancy can be distinguished according to the Gleason-Score Diagnosis. For this diagnosis a tissue sample is taken from the patient by biopsy and the morphology of the tissue is investigated by histological means. However, this approach mostly yields subjective results depending on the experience of the pathologist. To summarize, unfortunately, the diagnostic methods used so far are relatively insensitive and take the risk to yield false-positive results due to lack of specificity. Moreover, by using the current diagnostic methods any conclusions as regards the grade of malignancy, the progression of the tumor and its potential for metastasizing cannot be precisely predicted. Thus, the use of reliable diagnostic molecular markers would be highly beneficial for an understanding of the molecular basis of epithelial tumors, e.g. colon tumors, for distinguishing benign from malignant tissue and for grading and staging carcinomas, particularly for patients with metastasizing cancer having a very bad prognosis. It can be expected that such markers are also useful for the development of novel therapeutic avenues for cancer treatment.

The understanding of the molecular events underlying the transition of a normal cell into a tumor cell of different grades of aggressiveness and the availability of appropriate experimental systems to select for cancer-associated genes are absolute prerequisites for the identification of such novel diagnostic markers and therapeutic drug targets.

It is commonly accepted that tumorigenesis represents a complex multistage process in which genetic changes and environmental factors are thought to deregulate the cellular processes that control cell proliferation and differentiation. This multistep process is well illustrated for example by colorectal cancers, which typically develop over decades and appear to require multiple genetic events for completion (for review Kinzler and Vogelstein, 1996, Cell 87, 159-170). Both inheritance of altered genes (resulting in a marked predisposition) and genomic instability (caused by genotoxic agents from the environment) resulting in additional somatic mutations contribute to this process. Clearly, the list of decisive players causally involved in tumor formation is far from being complete and will obviously vary depending on the type of tumor.

Thus, the technical problem underlying the present invention is to provide means for diagnosis and therapy of epithelial tumors, which overcome the disadvantages of the presently available diagnostic and therapeutic methods.

The solution to said technical problem is achieved by providing the embodiments characterized in the claims.

The multistage process of chemically induced epithelial tumors in the skin was used as an experimental system to identify novel cancer-associated genes. Application of a single dose of DNA damaging substances onto the skin results in the formation of irreversible mutations in a few cells of the epidermis (keratinocytes), which will transform such cells into "silent" tumour-proned cells. As a result of subsequent application of substances, which by themselves do not induce damage DNA but enhance the frequency of tumour formation (so-called tumour promoters), non-malignant benign keratinocyte-derived tumors (papilloma) are formed followed by the formation of malignant and (with a low frequency) invasive carcinoma (Greenhalgh et al., 1990, Proc. Natl. Acad. Sci. USA 87:643-647; Yuspa et al. 1994, J Invest Dermatol 103, 90S-95S; Brown and Balmain (1995) Cancer Metastasis Rev 14,113-24).

During the experiments resulting in the present invention cDNAs and genomic sequences could be isolated encoding the TAP-70 protein presumably a member of the superfamily of aspartyl proteinases. High levels of transcripts of TAP-70 could be observed after phorbol ester treatment of murine skin and throughout the multistage carcinogenesis process in skin, relative to untreated control tissue. For determination of overexpression of the TAP-70 encoding gene in mouse skin tumors, slides with sections of tumors of various stages of progression (e.g. papilloma, tumors showing different degrees of differentiation etc.) were probed with an anti-TAP-70 in situ hybridisation probe and with a newly generated anti-TAP-70 antiserum. To monitor TAP-70 expression in human epithelial skin tumors, commercially available slides with sections of human tumors were probed with an anti-TAP-70 in situ hybridisation probe. The data obtained provide evidence that TAP-70 is involved in the process of skin carcinogenesis by supporting tumor cell growth and invasion. The new marker TAP-70 allows a better identification and classification of epithelial tumor cells (in particular colon, lung, breast, prostate, ovary, pancreas) and skin cancers with respect to proliferation, differentiation and malignancy. Based on the results of diagnosis an appropriate therapy can be applied. Based on the results obtained with other proteases involved in tumor development it has been seen that the modulation of TAP-70 gene expression and/or the overexpression, inhibition or reduction of the biological activity of the protein itself has a therapeutic effect. On the other hand, the TAP-70 protein (or the gene encoding it) can be regarded as a drug target allowing the identification of compounds useful for therapy.

**BRIEF DESCRIPTION OF THE DRAWINGS**

Figure 1: Nucleotide sequence (cDNA) and derived amino acid sequence of the human epithelial tumor associated TAP-70 polypeptide

Figure 2: Nucleotide sequence (cDNA) and derived amino acid sequence of the murine epithelial tumor associated TAP-70 polypeptide

Figure 3: Results of hybridization experiments relating to expression of TAP-70 in TPA-treated mouse skin **(A)**, in TPA-treated papillomas **(B)** and TPA-treated squamous cell carcinomas **(C)**
For experimental details see Example 1, below.

Figure 4: Results of Western Blots and in situ hybridization showing different expression of the TAP-70 encoding gene in the primary mouse keratinocyte cell line PMKR3 and mouse skin tumors

**(A)** Western Blot of PMKR3-extracts: upper part: Coomassie-stained SDS-PAGE gel; lower part: Western Blot using a polyclonal TAP-70 specific antibody (70)
**(B)** Immunohistochemical analysis of murine skin tumors confirming the results obtained by Western Blots; left panel: Results of incubation with anti-TAP-70 antiserum; right panel: PCNA-stained sections
**(C)** in situ hybridization with TAP-70 antisense specific probes on murine skin tumor sections.
For experimental details see Example 1,below

Figure 5. Study of the expression of TAP-70 in human tumors

**(A)** in situ hybridization using commercially available slides with human tissue samples;
**(B)** as (A) except that sections of skin tumors showing different degrees of malignancy were used;
**(C)** Northern Blot analyses, the band having a length of about 1,9 kb corresponds to TAP-70 mRNA;
**(D)** Results of Expression analyses of TAP-70 of various colon tumors.

Figure 6: Spatial structure of TAP-70 generated by homology modelling approaches

A homology model of TAP-70 was generated based on the crystal structure of the aspartic proteinase from Rhizomucor miehei (PDB-entry: 2asi) using the comparative modelling tool SWISS-MODEL. The template has been identified applying various sequence alignment programs (CLUSTAL), secondary prediction tools (Metapredict Protein Server, PHD) and searching in a database of protein families based on sequence and structural homology (HOMSTRAD).

The present invention relates to nucleic acid molecules encoding the mouse and human epithelial tumor associated polypeptides TAP-70 and especially polypeptides exhibiting a biological property of TAP-70 and being selected from the group consisting of

(a) a nucleic acid molecule encoding a polypeptide that comprises the amino acid sequence as depicted in Figure 1a or 2a;
(b) a nucleic acid molecule comprising the nucleotide sequence as depicted in Figure 1b or 2b;
(c) a nucleic acid molecule included in DSMZ Deposit No.

DSM 14829 (= plasmid 5'hTAP70)

DSM 14830 (= plasmid hcTAP70)

DSM 14831 (= plasmid 3'mTAP70)

DSM 14832 (= plasmid mgcTAP70)

(d) a nucleic acid molecule encoding a polypeptide the sequence of which shows at least 40% identity or more to the amino acid sequence of the polypeptide encoded by a nucleic acid molecule specified in (a) to (c);
(e) a nucleic acid molecule the sequence of which differs from the sequence of a nucleic acid molecule of (a) to (d) due to the degeneracy of the genetic code; and
(f) a nucleic acid molecule, which represents a fragment or allelic variant of a nucleic acid molecule of (a) to (e), and
(g) a nucleic acid, which encodes a fragment or a variant of the amino acid sequence depicted in Figure 2, which has an increased or decreased biological activity compared to the wild type amino acid sequence.

As used herein, a polypeptide exhibiting a biological property of TAP-70 is understood to be a polypeptide having at least one of the activities as illustrated in the Examples, below, i.e. at least one biological activity of an aspartyl proteinase, an activity in regulating cell proliferation, an acitivity in regulating cell differentiation or an activity in induction of tumors in mammals.
In a first embodiment, the invention provides an isolated nucleic acid molecule encoding a TAP-70 polypeptide that comprises the amino acid sequence as depicted in Figure 1a or 2a.
The present invention also provides a nucleic acid molecule encoding a TAP-70 polypeptide comprising the nucleotide sequence as depicted in Figure 1b or 2b.
The present invention provides not only the generated nucleotide sequence identified in Figure 1b and 2b, respectively and the predicted translated amino acid sequence, respectively, but also plasmid DNAs containing a TAP-70 cDNA (human and murine) deposited with the DSMZ (Deutsche Sammlung von Mikroorganismen und Zellkulturen GmbH, Mascheroder Weg 1b, 38124 Braunschweig, Germany), under

DSM 14829 (= plasmid 5'hTAP70)

DSM 14830 (= plasmid hcTAP70)

DSM 14831 (= plasmid 3'mTAP70)

DSM 14832 (= plasmid mgcTAP70)

on February 22, 2002.
The nucleotide sequence of each deposited TAP-70 clone can readily be determined by sequencing the deposited clone in accordance with known methods. The predicted amino acid sequence can then be verified from such deposits. Moreover, the amino acid sequence of the polypeptide encoded by each deposited clone can also be directly determined by peptide sequencing or by expressing the protein in a suitable host cell containing the deposited TAP-70 encoding DNA, collecting the protein, and determining its sequence.
The nucleic acid molecules of the invention can be both DNA and RNA molecules. Suitable DNA molecules are, for example, genomic or cDNA molecules. It is understood that all nucleic acid molecules encoding all or a portion of TAP-70 are also included, as long as they encode a polypeptide with the same biological activity as TAP70 or an inactive mutant thereof. The nucleic acid molecules of the invention can be isolated from natural sources or can be synthesized according to known methods.
The present invention also provides nucleic acid molecules encoding a polypeptide the amino acid sequence of which shows an identity of at least 40%, in particular an identity of at least 65% or 70%, preferably of at least 80% and, particularly preferred, of at least 83%, 85%, 86%, 87%, 88%, 89%, 90%, 91%, 92%, 93%, 94%, 95%, 96%, 97% or 98% to the amino acid sequence of the polypeptide of Figure 1 or 2. Such nucleic acid molecules are characterized by deletion, substitution and/or insertion of amino acid residue(s) compared to the amino acid sequences shown in Figure 1 or 2 or are the result of recombination. They can be naturally occurring variations, for example sequences from other organisms, or mutations that can either occur naturally or that have been introduced by specific mutagenesis. They can also be isolated, e.g., from genomic or cDNA libraries that were produced from human cell lines or tissues. In order to identify and isolate such nucleic acid molecules the molecules of the invention or parts of these molecules or the reverse complements of these molecules can be used, for example by means of hybridization. As a hybridization probe nucleic acid molecules can be used, for example, that have exactly or basically the nucleotide sequence as depicted in Figure 1 and 2, respectively, or parts of these sequences. The fragments used as hybridization probe can be synthetic fragments that were produced by means of conventional synthetic methods and the sequence of which basically corresponds to the sequence of a nucleic acid molecule of the invention. Furthermore nucleotide sequences - located in the noncoding regions of the genomic sequences of the disclosed TAP-70 nucleic acid may be used for generation of oligonucleotides for use as probes or primers.

As used herein, the term "hybridization" has the meaning of hybridization under conventional hybridization conditions, preferably under stringent conditions as described, for example, in Sambrook et al., Molecular Cloning, A Laboratory Manual 2$^{nd}$ edition (1989) Cold Spring Harbor Laboratory Press, Cold Spring Harbor, NY. However, in certain cases, a hybridizing nucleic acid molecule can also be detected at lower stringency hybridization conditions. Changes in the stringency of hybridization and signal detection are primarily accomplished through the manipulation of formamide concentration (lower percentages of formamide result in lowered stringency), salt conditions, or temperature. For example, lower stringency conditions include an overnight incubation at 37°Cin a solution comprising 6X SSPE (20X SSPE = 3M NaCl; 9.2M $NaH_2PO_4$; 0.02M EDTA, pH7.4), 0.5% SDS, 30% formamide, 100 µg/ml salmon sperm blocking DNA, following by washes at 50°C with 1 X SSPE, 0.1% SDS. In addition, to achieve even lower stringency, washes performed following stringent hybridization can be done at higher salt concentrations (e.g. 5X SSC). Variations in the above conditions may be accomplished through the inclusion and/or substitution of alternate blocking reagents used to suppress background in hybridization experiments. The inclusion of specific blocking reagents may require modification of the hybridization conditions described above, due to problems with compatibility.

The nucleic acid molecules of the present invention also include molecules which differ from the nucleic acid molecules with sequences shown in Figures 1 and 2 due to the degeneracy of the genetic code.

In a further embodiment, the present invention provides nucleic acid molecules which comprise fragments or allelic variants of the nucleic acid molecules described above encoding a polypeptide of the invention. "Fragments" are understood to be parts of the nucleic acid molecules that are long enough to encode one of the described polypeptids. These fragments also comprise nucleic acid molecules specifically hybridizing to transcripts of the nucleic acid molecules of the invention. These nucleic acid molecules can be used, for example, as probes or primers in the diagnostic assay and/or kit described below and, preferably, are oligonucleotides having a length of at least 10, in particular of at least 15 and particularly preferred of at least 50 nucleotides. The nucleic acid molecules and oligonucleotides of the invention can also be used, for example, as primers for a nucleic acid based amplification reaction such as for example polymerase chain reaction.

The allelic variants can be either naturally occurring variants or synthetically produced variants or variants produced by recombinant DNA processes.

Generally, by means of conventional molecular biological processes it is possible (see, e.g., Sambrook et al., supra) to introduce different mutations into the nucleic acid molecules of the invention. As a result TAP-70 polypeptides or TAP-70 related polypeptids with possibly modified biological properties are synthesized. One possibility is the production of deletion mutants in which nucleic acid molecules are produced by continuous deletions from the 5'- or 3'-terminal of the coding DNA sequence and that lead to the synthesis of polypeptids that are shortened accordingly. Another possibility is the introduction of single-point mutation at positions where a modification of the amino aid sequence influences, e.g., the proteolytic properties. By this method muteins can be produced, for example, that possess a modified $K_m$-value or that are no longer subject to the regulation mechanisms that normally exist in the cell, e.g. with regard to allosteric regulation or covalent modification. Such muteins might also be valuable as therapeutically useful antagonists of TAP-70.

For the manipulation in prokaryotic cells by means of genetic engineering the nucleic acid molecules of the invention or parts of these molecules can be introduced into plasmids allowing a mutagenesis or a modification of a sequence by recombination of DNA sequences. By means of conventional methods (cf. Sambrook et al., supra) bases can be exchanged and natural or synthetic sequences can be added. In order to link the DNA fragments with each other adapters or linkers can be added to the fragments. Furthermore, manipulations can be performed that provide suitable cleavage sites or that remove superfluous DNA or cleavage sites. If insertions, deletions or substitutions are possible, in vitro mutagenesis, primer repair, restriction or ligation can be performed. As analysis method usually sequence analysis, restriction analysis and other biochemical or molecular biological methods are used.

The polypeptids encoded by the various variants of the nucleic acid molecules of the invention show certain common characteristics, such as proteolytic activity, activity in the regulation of cell proliferation and differentiation, molecular weight, immunological reactivity or conformation or physical properties like the electrophoretical mobilty, chromatographic behavior, sedimentation coefficients, solubility, spectroscopic properties, stability, pH optimum, temperature optimum.

The invention furthermore relates to vectors containing the _nucleic acid molecules of the invention. Preferably, they are plasmids, cosmids, viruses, bacteriophages and other vectors usually used in the field of genetic engineering. Vectors suitable for use in the present invention include, but are not limited to the T7-based dual expression vectors (expression in prokaryotes and in eucaryotes) for expression in mammalian cells and baculovirus-derived vectors for expression in insect cells. Preferably, the nucleic acid molecule of the invention is operatively linked to the regulatory elements in the recombinant vector of the invention that guarantee the transcription and synthesis of an mRNA in prokaryotic and/or eukaryotic cells that can be translated. The nucleotide sequence to be transcribed can be operably linked to a promoter like a T7, metallothionein I or polyhedrin promoter.

In a further embodiment, the present invention relates to recombinant host cells transiently or stably containing the nucleic acid molecules or vectors or the invention. A host cell is understood to be an organism that is capable to take up *in vitro* recombinant DNA and, if the case may be, to synthesize the polypeptids encoded by the nucleic acid molecules of the invention. Preferably, these cells are prokaryotic or eukaryotic cells, for example mammalian cells, bacterial cells, insect cells or yeast cells. The host cells of the invention are preferably characterized by the fact that the introduced nucleic acid molecule of the invention either is heterologous with regard to the transformed cell, i.e. that it does not naturally occur in these cells, or is localized at a place in the genome different from that of the corresponding naturally occurring sequence.

The present invention also relates to an isolated epithelial tumor associated TAP-70 polypeptide or a polypeptide exhibiting a biological property of the human epithelial tumor associated polypeptide TAP-70 being selected from a group consisting of

(a) a polypeptide, which is encoded by a nucleic acid molecule of claim 1.
(b) a polypeptide, which comprises an amino acid sequence given in Figure 1 or 2;
(c) a polypeptide, that is recognized by a binding agent, that has been raised against and is specifically binding the polypeptide of (a) or (b);
(d) a fragment or a variant of the polypeptides of (a)-(c), that is encoded by a nucleic acid sequence, that hybridizes to a nucleic acid according to claim 1 under stringent conditions; and
(e) a fragment or variant of the polypeptides of (a)-(d), which has an increased or decreased biological activity compared to the wild type TAP-70 polypeptide.

A further embodiment of the invention relates to a polypeptide exhibiting a biological property of the human epithelial tumor associated polypeptide TAP-70 and being encoded by the nucleic acid molecules of the invention, as well as to methods for their production, whereby, e.g., a host cell of the invention is cultivated under conditions allowing the synthesis of the polypeptide and the polypeptide is subsequently isolated from the cultivated cells and/or the culture medium. Isolation and purification of the recombinantly produced polypeptide may be carried out by conventional means including preparative chromatography and affinity and immunological separations using, e.g., an anti-TAP-70 antibody, or, e.g., can be substantially purified by the one-step method described in Smith and Johnson, Gene 67; 31-40 (1988). These polypeptides, however, not only comprise recombinantly produced polypeptides but include isolated naturally occurring polypeptides, synthetically produced polypeptides, or polypeptides produced by a combination of these methods. Means for preparing such polypeptides or related polypeptides are well understood in the art. These polypeptides are preferably in a substantially purified form.

The polypeptides according to the present invention comprise also polypeptides that are fusion or chimeric polypeptides comprising the amino acid sequence encoded by the nucleic acid sequence of the TAP 70 disclosed herein. The polypeptides may be fused to any suitable amino acid sequences. These sequences may for example comprise antigenic fragments, receptors, enzymes, toxins, chelating epitopes,etc.. In a preferred embodiment of the present invention the amino acid sequences, that are fused to the disclosed polypeptides are tags useful in the purification or recovery of the polypeptides such as e.g. his-tags or myc-tags. The amino acid sequences fused together may be directly linked or may be separated by any linker or spacer sequences suitable in the particular purpose.

The present invention also relates to binding agents, that bind specifically to a TAP-70 polypeptide or a related polypeptide as defined above. The term binding agent comprises a variety of substances such as oligopeptides, antibodies, peptdiomimetic molecules comprising antigen binding oligopeptides, nucleic acids, carbohydrates, organic compounds, etc.. Antibody according to the present invention preferably relates to antibodies which consist essentially of pooled monoclonal antibodies with different epitopic specifities, as well as distinct monoclonal antibody preparations. Monoclonal antibodies are made from an antigen containing fragments of the polypeptides of the invention by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab') 2 fragments) which are capable of specifically binding to protein. Fab and f(ab')2 fragments lack the Fc fragment of intact antibody, clear more rapidly from the circulation, and may have less non-specific tissue binding than an intact antibody. (Wahl et al., J. Nucl. Med. 24: 316-325 (1983)). Thus, these fragments are preferred, as well as the products of a FAB or other immunoglobulin expression library. Moreover, antibodies of the present invention include chimerical, single chain, and humanized antibodies.

Binding agents according to the present invention may for example be employed for the inhibition of the activity of the TAP-70 polypeptides disclosed herein. In this respect the term "binding agents" relates to agents specifically binding to the polypeptides transcribed from the novel TAP-70 nucleic acids and thus inhibiting the activity of said polypeptide. Such binding agents may for example comprise nucleic acids (DNA, RNA, PNA etc.), polypeptides (antibodies, receptors, antigenic fragments, oligopeptides), carbohydrates, lipids, organic or inorganic compounds (metal-ions, sulfur compounds, boranes, silicates, reducing agents, oxidizing agents). The binding agents may preferably interact with the polypeptide by binding to epitopes, that are essential for the biological activity. The interaction may be reversible or irreversibly. The binding may be noncovalent or even covalent binding to the polypeptide. Furthermore the binding agents may introduce alterations to the polypeptide, that alter or diminish the biological activity of the inventive polypeptide.

For certain purposes, e.g. diagnostic methods, the antibody of the present invention can be detectably labeled, for example, with a radioisotope, a bioluminescent compound, a chemiluminescent compound, a fluorescent compound, a metal chelate, or an enzyme. Furthermore any method suitable for the detection of the intermolecular interaction may be employed.

The invention also relates to a transgenic non-human animal such as transgenic mouse, rats, hamsters, dogs, monkeys, rabbits, pigs, C. elegans and fish such as torpedo fish comprising a nucleic acid molecule or vector of the invention, preferably wherein said nucleic acid molecule or vector may be stably integrated into the genome of said non-human animal, preferably such that the presence of said nucleic acid molecule or vector leads to the expression of the TAP-70 polypeptide (or related polypeptide) of the invention, or may otherwise be transiently expressed within the non-human animal. Said animal may have one or several copies of the same or different nucleic acid molecules encoding one or several forms of TAP-70 polypeptide or mutant forms thereof. This animal has numerous utilities, including as a research model for function of an aspartyl proteinases involved in the regulation of cell proliferation and differentiation and therefore, presents a novel and valuable animal in the development of therapies, treatment, etc. for diseases caused by deficiency or failure of aspartyl proteinases involved in the development of cell proliferative disorders, e.g., epithelial tumors. Accordingly, in this instance, the non-human mammal is preferably a laboratory animal such as a mouse or rat.

Preferably, the transgenic non-human animal of the invention further comprises at least one inactivated wild type allele of the corresponding TAP-70 encoding gene. This embodiment allows for example the study of the interaction of various mutant forms of TAP-70 polypeptides on the onset of the clinical symtoms of disease related to disorders in the metabolism of aspartyl proteinases involved in the regulation of cell proliferation and differentiation. All the applications that have been herein before discussed with regard to a transgenic animal also apply to animals carrying two, three or more transgenes. It might be also desirable to inactivate TAP-70 protein expression or function at a certain stage of development and/or life of the transgenic animal. This can be achieved by using, for example, tissue specific, developmental and/or cell regulated and/or inducible promoters which drive the expression of, e.g., an antisense or ribozyme directed against the RNA transcript encoding the TAP-70 encoding mRNA; see also supra. A suitable inducible system is for example tetracycline-regulated gene expression as described, e.g., by Gossen and Bujard (Proc. Natl. Acad. Sci. 89 USA (1992), 5547-5551) and Gossen et al. (Trends Biotech. 12 (1994), 58-62). Similar, the expression of the mutant TAP-70 protein may be controlled by such regulatory elements.

Furthermore, the invention also relates to a transgenic mammalian cell which contains (preferably stably integrated into its genome or transiently introduced) a nucleic acid molecule according to the invention or part thereof, wherein the transcription and/or expression of the nucleic acid molecule or part thereof leads to reduction of the synthesis of a TAP-70 protein. In a preferred embodiment, the reduction is achieved by an anti-sense, sense, ribozyme, co-suppression and/or dominant mutant effect. "Antisense" and "antisense nucleotides" means DNA or RNA constructs which block the expression of the naturally occurring gene product. In another preferred embodiment the native nucleic acid sequence coding for the TAP-70 polypeptide may be altered or substituted by a variant of said nucleic acid sequence, e.g. by means of recombination, thus rendering the TAP-70 gene non functional. Thus an organism lacking the TAP-70 polypeptide activity may be produced according to knock out experiments.

The provision of the nucleic acid molecule according to the invention opens up the possibility to produce transgenic non-human animals with a reduced level of the TAP-70 protein as described above and, thus, with a defect in metabolism of aspartyl proteinases involved in the regulation of cell proliferation and differentiation. Techniques how to achieve this are well known to the person skilled in the art. These include, for example, the expression of antisense-RNA, ribozymes, of molecules which combine antisense and ribozyme functions and/or of molecules which provide for a co-suppression effect. When using the antisense approach for reduction of the amount of TAP-70 proteins in cells, the nucleic acid molecule encoding the antisense-RNA is preferably of homologous origin with respect to the animal species used for transformation. However, it is also possible to use nucleic acid molecules which display a high degree of homology to endogenously occurring nucleic acid molecules encoding a TAP-70 protein. In this case the homology is preferably higher than 80%, particularly higher than 90% and still more preferably higher than 95%. The reduction of the synthesis of a polypeptide according to the invention in the transgenic mammalian cells can result in an alteration in, e.g., degradation of endogenous proteins. In transgenic animals comprising such cells this can lead to various physiological, developmental and/or morphological changes.

Thus, the present invention also relates to transgenic non-human animals comprising the above-described transgenic cells. These may show, for example, a deficiency in protein degradation compared to wild type animals due to the stable or transient presence of a foreign DNA resulting in at least one of the following features:

(a) disruption of (an) endogenous gene(s) encoding TAP-70;
(b) expression of at least one antisense RNA and/or ribozyme against a transcript comprising a nucleic acid molecule of the invention;
(c) expression of a sense and/or non-translatable mRNA of the nucleic acid molecule of the invention;
(d) expression of an antibody of the invention;
(e) incorporation of a functional or non-functional copy of the regulatory sequence of the invention; or
(f) incorporation of a recombinant DNA molecule or vector of the invention.

Methods for the production of a transgenic non-human animal of the present invention, preferably transgenic mouse, are well known to the person skilled in the art. Such methods, e.g., comprise the introduction of a nucleic acid molecule or vector of the invention into a germ cell, an embryonic cell, stem cell or an egg or a cell derived therefrom. The non-human animal can be used in accordance with a screening method of the invention described herein and may be a non-transgenic healthy animal, or may have a disorder, preferably a disorder caused by at least one mutation in the TAP-70 protein. Such transgenic animals are well suited for, e.g., pharmacological studies of drugs in connection with mutant forms of the above described TAP-70 polypeptide. Production of transgenic embryos and screening of those can be performed, e.g., as described by A. L. Joyner Ed., Gene Targeting, A Practical Approach (1993), Oxford University Press. The DNA of the embryonal membranes of embryos can be analyzed using, e.g., Southern blots with an appropriate probe, amplification techniques based on nucleic acids (e.g. PCR) etc.; see supra.

In a further aspect, the present invention, relates to a method for identifying a binding partner to a TAP-70 polypeptide (or related polypeptide) of the invention comprising:

(a) contacting a TAP-70 polypeptide of the invention with a compound to be screened; and
(b) determining whether the compound effects an activity of the polypeptide.

TAP-70 polypeptides may be used to screen for proteins or other compounds that bind to TAP-70 or for proteins or other compounds to which TAP-70 binds. The binding of TAP-70 and the molecule may activate (agonist), increase, inhibit (antagonist), or decrease activity of the TAP-70 or the molecule bound. Examples of such molecules include antibodies, oligonucleotides, proteins (e.g., receptors), or small molecules.

Preferably, the molecule is closely related to the natural ligand of TAP-70, e.g., a fragment of the ligand, or a natural substrate, a ligand, a structural or functional mimetic; see, e.g., Coligan, Current Protocols in Immunology 1(2) (1991); Chapter 5. Similarly, the molecule can be closely related to the natural receptor to which TAP-70 might bind, or at least, a fragment of the receptor capable of being bound by TAP-70 (e.g., active site). In either case, the molecule can be rationally designed using known techniques.

Preferably, the screening for these molecules involves producing appropriate cells which express TAP-70, either as a secreted protein or on the cell membrane. Preferred cells include cells from mammals, yeast, Drosophila, or E. coli. Cells expressing TAP-70 (or cell membrane containing the expressed polypeptide) are then preferably contacted with a test compound potentially containing the molecule to observe binding, stimulation, or inhibition of activity of TAP-70.

The assay may simply test binding of a candidate compound to TAP-70, wherein binding is detected by a label, or in an assay involving competition with a labeled competitor. Further, the assay may test whether the candidate compound results in a signal generated by binding to TAP-70.

Alternatively, the assay can be carried out using cell-free preparations, polypeptide/molecule affixed to a solid support, chemical libraries, or natural product mixtures. The assay may also simply comprise the steps of mixing a candidate compound with a solution containing TAP-70, measuring TAP-70/molecule activity or binding, and comparing the TAP-70/molecule activity or binding to a standard.

Preferably, an ELISA assay can measure TAP-70 level or activity in a sample (e.g., biological sample) using a monoclonal or polyclonal antibody. The antibody can measure TAP-70 level or activity by either binding, directly or indirectly, to TAP-70 or by competing with TAP-70 for a substrate. All of these above assays can be used as diagnostic or prognostic markers. The molecules discovered using these assays can be used to treat disease or to bring about a particular result in a patient (e.g., elimination of a epthelial tumor or stop of progression of tumor growth) by activating or inhibiting the TAP-70 molecule. Moreover, the assays can discover agents which may inhibit or enhance the production of TAP-70 from suitably manipulated cells or tissues.

Therefore, the invention includes a method of identifying compounds which bind to a TAP-70 polypeptide comprising the steps of: (a) incubating a candidate binding compound with a polypeptide of the invention(TAP-70); and (b) determining if binding has occurred.

Moreover, the invention includes a method of identifying activators/agonists or inhibitors/antagonists of a TAP-70 polypeptide comprising the steps of: (a) incubating a candidate compound with a polypeptide of the invention; b) assaying a biological activity, and (c) determining if a biological activity of the polypeptide of the invention has been altered.

Furthermore, the present invention relates to a method for identifying activators or inhibitors of the expression of TAP-70 polypeptide comprising the steps of:

(a) incubating a candidate compound with an in-vivo or in-vitro test system for protein expression or administering a compound to a test organism,
(b) detecting the level of the TAP 70 polypeptide within the test system or within the organism, and
(c) determining if the level of said polypeptide has been altered.

In a further embodiment, the present invention relates to method of identifying and obtaining a drug candidate for therapy of a epithelial tumor comprising the steps of

(a) contacting a TAP-70 polypeptide of the present invention or a cell expressing said polypeptide in the presence of components capable of providing a detectable signal in response

  i. to protein degradation
  ii. to altered regulation of cell proliferation
  iii. to altered cell differentiation, with said drug candidate to be screened under conditions to allow protein degradation, and

(b) detecting presence or absence of a signal or increase of the signal generated from protein degradation, wherein the presence or increase of the signal is indicative for a putative drug.

For example, an assay employing the degradation of hemoglobin can be used to measure TAP-70 dependent protein degradation. Furthermore experiments using animals or isolated cells or cell lines may be used to examine the proliferative behavior of cells or tissues in dependence on the TAP-70 action. The same procedures may be employed for the study of cell differentiation.

The drug candidate may be a single compound or a plurality of compounds. The term "plurality of compounds" in a method of the invention is to be understood as a plurality of substances which may or may not be identical.

Said compound or plurality of compounds may be chemically synthesized or microbiologically produced and/or comprised in, for example, samples, e.g., cell extracts from, e.g., plants, animals or microorganisms. Furthermore, said compound(s) may be known in the art but hitherto not known to be capable of suppressing or activating TAP-70 polypeptides. The reaction mixture may be a cell free extract or may comprise a cell or tissue culture. Suitable set ups for the method of the invention are known to the person skilled in the art and are, for example, generally described in Alberts et al., Molecular Biology of the Cell, third edition (1994) and in the appended examples. The plurality of compounds may be, e.g., added to the reaction mixture, culture medium, injected into a cell or otherwise applied to the transgenic animal. The cell or tissue that may be employed in the method of the invention preferably is a host cell, mammalian cell or non-human transgenic animal of the invention described in the embodiments hereinbefore.

If a sample containing a compound or a plurality of compounds is identified in the method of the invention, then it is either possible to isolate the compound from the original sample identified as containing the compound capable of suppressing or activating TAP-70, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical.

Several methods are known to the person skilled in the art for producing and screening large libraries to identify compounds having specific affinity for a target. These methods include the phage-display method in which randomized peptides are displayed from phage and screened by affinity chromatography to an immobilized receptor; see, e.g., WO 91/17271, WO 92/01047, US-A-5,223,409. In another approach, combinatorial libraries of polymers immobilized on a chip are synthesized using photolithography; see, e.g., US-A-5,143,854, WO 90/15070 and WO 92/10092. The immobilized polymers are contacted with a labeled receptor and scanned for label to identify polymers binding to the receptor. The synthesis and screening of peptide libraries on continuous cellulose membrane supports that can be used for identifying binding ligands of the polypeptide of the invention and thus possible inhibitors and activators is described, for example, in Kramer, Methods Mol. Biol. 87 (1998), 25-39. This method can also be used, for example, for determining the binding sites and the recognition motifs in the polypeptide of the invention. In like manner, the substrate specificity of the DnaK chaperon was determined and the contact sites between human interleukin-6 and its receptor; see Rudiger, EMBO J. 16 (1997), 1501-1507 and Weiergraber, FEBS Lett. 379 (1996), 122-126, respectively. Furthermore, the above-mentioned methods can be used for the construction of binding supertopes derived from the polypeptide of the invention. A similar approach was successfully described for peptide antigens of the anti-p24 (HIV-1) monoclonal antibody; see Kramer, Cell 91 (1997), 799-809. A general route to fingerprint analyses of peptide-antibody interactions using the clustered amino acid peptide library was described in Kramer, Mol. Immunol. 32 (1995), 459-465. In addition, antagonists of the TAP-70 polypeptide of the invention can be derived and identified from monoclonal antibodies that specifically react with the polypeptide of the invention in accordance with the methods as described in Doring, Mol. Immunol. 31 (1994), 1059-1067.

More recently, WO 98/25146 described further methods for screening libraries of complexes for compounds having a desired property, especially, the capacity to agonize, bind to, or antagonize a polypeptide or its cellular receptor. The complexes in such libraries comprise a compound under test, a tag recording at least one step in synthesis of the compound, and a tether susceptible to modification by a reporter molecule. Modification of the tether is used to signify that a complex contains a compound having a desired property. The tag can be decoded to reveal at least one step in the synthesis of such a compound. Other methods for identifying compounds which interact with the polypeptides according to the invention or nucleic acid molecules encoding such molecules are, for example, the in vitro screening with the phage display system as well as filter binding assays or "real time" measuring of interaction using, for example, the BIAcore apparatus (Pharmacia).

All these methods can be used in accordance with the present invention to identify activators/agonists and inhibitors/antagonists of the TAP-70 polypeptide or related polypeptide of the invention.

Various sources for the basic structure of such an activator or inhibitor can be employed and comprise, for example, mimetic analogs of the polypeptide of the invention. Mimetic analogs of the polypeptide of the invention or biologically active fragments thereof can be generated by, for example, substituting the amino acids that are expected to be essential for the biological activity with, e.g., stereoisomers, i.e. D-amino acids; see e.g., Tsukida, J. Med. Chem. 40 (1997), 3534-3541. Furthermore, in case fragments are used for the design of biologically active analogs pro-mimetic components can be incorporated into a peptide to reestablish at least some of the conformational properties that may have been lost upon removal of part of the original polypeptide; see, e.g., Nachman, Regul. Pept. 57 (1995), 359-370. Furthermore, the TAP-70 polypeptide of the invention can be used to identify synthetic chemical peptide mimetics that bind to or can function as a ligand, substrate, binding partner or the receptor of the polypeptide of the invention as effectively as does the natural polypeptide; see, e.g., Engleman, J. Clin. Invest. 99 (1997), 2284-2292. For example, folding simulations and computer redesign of structural motifs of the polypeptide of the invention can be performed using appropriate computer programs (Olszewski, Proteins 25 (1996), 286-299; Hoffman, Comput. Appl. Biosci. 11 (1995), 675-679). Computer modeling of protein folding can be used for the conformational and energetic analysis of detailed peptide and protein models (Monge, J. Mol. Biol. 247 (1995), 995-1012; Renouf, Adv. Exp. Med. Biol. 376 (1995), 37-45). In particular, the appropriate programs can be used for the identification of interactive sites of the TAP-70 polypeptide and its possible receptor, its ligand or other interacting proteins by computer assistant searches for complementary peptide sequences (Fassina, Immunomethods 5 (1994), 114-120. Further appropriate computer systems for the design of protein and peptides are described in the prior art, for example in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used for, e.g., the preparation of peptide mimetics of the protein of the invention or fragments thereof. Such pseudopeptide analogues of the natural amino acid sequence of the protein may very efficiently mimic the parent protein (Benkirane, J. Biol. Chem. 271 (1996), 33218-33224). For example, incorporation of easily available achiral ω-amino acid residues into a protein of the invention or a fragment thereof results in the substitution of amide bonds by polymethylene units of an aliphatic chain, thereby providing a convenient strategy for constructing a peptide mimetic (Banerjee, Biopolymers 39 (1996), 769-777). Superactive peptidomimetic analogues of small peptide hormones in other systems are described in the prior art (Zhang, Biochem. Biophys. Res. Commun. 224 (1996), 327-331). Appropriate peptide mimetics of the protein of the present invention can also be identified by the synthesis of peptide mimetic combinatorial libraries through successive amide alkylation and testing the resulting compounds, e.g., for their binding and immunological properties. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, a three-dimensional and/or crystallographic structure of the polypeptide of the invention can be used for the design of peptide mimetic inhibitors of the biological activity of the polypeptide of the invention (Rose, Biochemistry 35 (1996), 12933-12944; Rutenber, Bioorg. Med. Chem. 4 (1996), 1545-1558).

The structure-based design and synthesis of low-molecular-weight synthetic molecules that mimic the activity of the native biological polypeptide is further described in, e.g., Dowd, Nature Biotechnol. 16 (1998), 190-195; Kieber-Emmons, Current Opinion Biotechnol. 8 (1997), 435-441; Moore, Proc. West Pharmacol. Soc. 40 (1997), 115-119; Mathews, Proc. West Pharmacol. Soc. 40 (1997), 121-125; Mukhija, European J. Biochem. 254 (1998), 433-438.

It is also well known to the person skilled in the art, that it is possible to design, synthesize and evaluate mimetics of small organic compounds that, for example, can act as a substrate or ligand to the TAP-70 polypeptide of the invention or the related polypeptide. For example, it has been described that D-glucose mimetics of hapalosin exhibited similar efficiency as hapalosin in antagonizing multidrug resistance assistance-associated protein in cytotoxicity; see Dinh, J. Med. Chem. 41 (1998), 981-987.

The nucleic acid molecule of the invention can also serve as a target for activators and inhibitors. Activators may comprise, for example, proteins that bind to the mRNA of a gene encoding a TAP-70 polypeptide of the invention, thereby stabilizing the native conformation of the mRNA and facilitating transcription and/or translation, e.g., in like manner as Tat protein acts on HIV-RNA. Furthermore, methods are described in the literature for identifying nucleic acid molecules such as an RNA fragment that mimics the structure of a defined or undefined target RNA molecule to which a compound binds inside of a cell resulting in retardation of cell growth or cell death; see, e.g., WO 98/18947 and references cited therein. These nucleic acid molecules can be used for identifying unknown compounds of pharmaceutical and/or agricultural interest, and for identifying unknown RNA targets for use in treating a disease. These methods and compositions can be used in screening for novel antibiotics, bacteriostatics, or modifications thereof or for identifying compounds useful to alter expression levels of proteins encoded by a nucleic acid molecule. Alternatively, for example, the conformational structure of the RNA fragment which mimics the binding site can be employed in rational drug design to modify known antibiotics to make them bind more avidly to the target. One such methodology is nuclear magnetic resonance (NMR), which is useful to identify drug and RNA conformational structures. Still other methods are, for example, the drug design methods as described in WO 95/35367, US-A-5,322,933, where the crystal structure of the RNA fragment can be deduced and computer programs are utilized to design novel binding compounds which can act as antibiotics.

Some genetic changes lead to altered protein conformational states. For example, some mutant TAP-70 polypetides may possess a tertiary structure that renders them far less capable of protein degradation. Restoring the normal or regulated conformation of mutated proteins is the most elegant and specific means to correct these molecular defects, although it may be difficult. Of particular interest in this regard is the consensus domain of TAP-70 described in the examples, below. Pharmacological manipulations thus may aim at restoration of wild-type conformation of the TAP-70 poylpeptide. Thus, the nucleic acid molecules and encoded polypeptides of the present invention may also be used to design and/or identify molecules which are capable of activating the wild-type, i.e. "TAP-70" or "anti-TAP-70" function of a TAP-70 polypeptide or related polypepetide.

The compounds which can be tested and identified according to a method of the invention may be expression libraries, e.g., cDNA expression libraries, peptides, proteins, nucleic acids, antibodies, small organic compounds, hormones, peptidomimetics, PNAs or the like (Milner, Nature Medicine 1 (1995), 879-880; Hupp, Cell 83 (1995), 237-245; Gibbs, Cell 79 (1994), 193-198 and references cited supra). Furthermore, genes encoding a putative regulator of TAP-70 polypeptide and/or which excert their effects up- or downstream the TAP-70 polypeptide of the invention may be identified using, for example, insertion mutagenesis using, for example, gene targeting vectors known in the art. Said compounds can also be functional derivatives or analogues of known inhibitors or activators. Such useful compounds can be for example transacting factors which bind to the TAP-70 polypeptide or regulatory sequences of the gene encoding it. Identification of transacting factors can be carried out using standard methods in the art (see, e.g., Sambrook, supra, and Ausubel, supra). To determine whether a protein binds to the protein itself or regulatory sequences, standard native gel-shift analyses can be carried out. In order to identify a transacting factor which binds to the protein or regulatory sequence, the protein or regulatory sequence can be used as an affinity reagent in standard protein purification methods, or as a probe for screening an expression library. The identification of nucleic acid molecules which encode polypeptides which interact with the TAP-70 polypeptids described above can also be achieved, for example, as described in Scofield (Science 274 (1996), 2063-2065) by use of the so-called yeast "two-hybrid system". In this system the polypeptide encoded by a nucleic acid molecule according to the invention or a smaller part thereof is linked to the DNA-binding domain of the GAL4 transcription factor. A yeast strain expressing this fusion polypeptide and comprising a lacZ reporter gene driven by an appropriate promoter, which is recognized by the GAL4 transcription factor, is transformed with a library of cDNAs which will express plant proteins or peptides thereof fused to an activation domain. Thus, if a peptide encoded by one of the cDNAs is able to interact with the fusion peptide comprising a peptide of a TAP-70 polypeptide of the invention, the complex is able to direct expression of the reporter gene. In this way the nucleic acid molecules according to the invention and the encoded peptide can be used to identify peptides and proteins interacting with TAP-70 protein. It is apparent to the person skilled in the art that this and similar systems may then further be exploited for the identification of inhibitors of the binding of the TAP-70 proteins.

Once the transacting factor is identified, modulation of its binding to or regulation of expression of the TAP-70 polypeptide of the invention can be pursued, beginning with, for example, screening for inhibitors against the binding of the transacting factor to the protein of the present invention. Activation or repression of TAP-70 proteins could then be achieved in animals by applying the transacting factor (or its inhibitor) or the gene encoding it, e.g. in an expression vector. In addition, if the active form of the transacting factor is a dimer, dominant-negative mutants of the transacting factor could be made in order to inhibit its activity. Furthermore, upon identification of the transacting factor, further components in the pathway leading to activation (e.g. signal transduction) or repression of a gene involved in the control of TAP-70 then can be identified. Modulation of the activities of these components can then be pursued, in order to develop additional drugs and methods for modulating the metabolism of protein degradation in animals. Thus, the present invention also relates to the use of the two-hybrid system as defined above for the identification of TAP-70 or activators or inhibitors of TAP-70.

The compounds isolated by the above methods also serve as lead compounds for the development of analog compounds. The analogs should have a stabilized electronic configuration and molecular conformation that allows key functional groups to be presented to the TAP-70 or its possible receptor in substantially the same way as the lead compound. In particular, the analog compounds have spatial electronic properties which are comparable to the binding region, but can be smaller molecules than the lead compound, frequently having a molecular weight below about 2 kD and preferably below about 1 kD. Identification of analog compounds can be performed through use of techniques such as self-consistent field (SCF) analysis, configuration interaction (CI) analysis, and normal mode dynamics analysis. Computer programs for implementing these techniques are available; e.g., Rein, Computer-Assisted Modeling of Receptor-Ligand Interactions (Alan Liss, New York, 1989). Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art; see also supra. Furthermore, peptidomimetics and/or computer aided design of appropriate derivatives and analogues can be used, for example, according to the methods described above.

In a preferred embodiment of the above-described methods of the invention said cell is a cell of or, obtained by a method of the invention or is comprised in the above-described transgenic non-human animal.

Once the described compound has been identified and obtained, it is preferably provided in a therapeutically acceptable form.

Accordingly, the present invention also relates to a pharmaceutical composition comprising a nucleic acid molecule, polypeptide, recombinant vector, antibody, activator/agonist, inhibitor/antagonist and/or binding partner according to the present invention and a pharmaceutically acceptable excipient, diluent or carrier.

In a particular embodiment the TAP 70 polypeptide, the - recombinant vector, the antisense RNA, the ribozyme, the binding agent or the identified activator/agonist, inhibitor/antagonist or binding partner are used for the preparation of a medicament for treatment of disorders associated with a non wild-type expression of the TAP-70 molecules.

Examples of suitable pharmaceutical carriers etc. are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Administration of the suitable compositions may be effected by different ways, e.g. by intravenous, intraperetoneal, subcutaneous, intramuscular, topical or intradermal administration. The route of administration, of course, depends on the nature of the tumor, its localisation and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind and stage of the tumor, general health and other drugs being administered concurrently.

The delivery of the nucleic acid molecules of the invention can be achieved by direct application or, preferably, by using a recombinant expression vector such as a chimeric virus containing these compounds or a colloidal dispersion system. Direct application to the target site can be performed, e.g., by ballistic delivery, as a colloidal dispersion system or by catheter to a site in artery. The colloidal dispersion systems which can be used for delivery of the above nucleic acid molecules include macromolecule complexes, nanocapsules, microspheres, beads and lipid-based systems including oil-in-water emulsions (mixed), micelles, liposomes and lipoplexes, The preferred colloidal system is a liposome. The composition of the liposome is usually a combination of phospholipids and steroids, especially cholesterol. The skilled person is in a position to select such liposomes which are suitable for the delivery of the desired nucleic acid molecule. Organ-specific or cell-specific liposomes can be used in order to achieve delivery only to the desired tumor. The targeting of liposomes can be carried out by the person skilled in the art by applying commonly known methods. This targeting includes passive targeting (utilizing the natural tendency of the liposomes to distribute to cells of the RES in organs which contain sinusoidal capillaries) or active targeting (for example by coupling the liposome to a specific ligand, e.g., an antibody, a receptor, sugar, glycolipid, protein etc., by well known methods). In the present invention monoclonal antibodies are preferably used to target liposomes to specific tumors via specific cell-surface ligands.

Preferred recombinant vectors useful for gene therapy are viral vectors, e.g. adenovirus, herpes virus, vaccinia, or, more preferably, an RNA virus such as a Retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

In order to achieve expression only in the target organ, e.g., an epithelial tumor to be treated, the nucleic acid molecules of the present invention can be linked to a tissue specific promoter and used for gene therapy. Such promoters are well known to those skilled in the art (see e.g. Zimmermann et al., (1994) Neuron 12, 11-24; Vidal et al.; (1990) EMBO J. 9, 833-840; Mayford et al., (1995), Cell 81, 891-904; Pinkert et al., (1987) Genes & Dev. 1, 268-76).

The present invention also relates to the use of the above compounds of the invention for the preparation of a pharmaceutical composition for treatment of an epithelial tumor, preferably a colon tumor.

The present invention also relates to a method for detecting cells expressing a TAP-70 molecule (polypeptide or nucleic acid) encoded by the novel TAP-70 disclosed herein. The cells expressing the novel TAP-70 polypeptide may for example comprise neoplastic -cells, tumor cells, precursor cells of -tumors or, cells showing a disposition to a tumor.

Thus, the present invention relates to a method of diagnosing tumors and especially epithelial tumors or a susceptibility to tumors such as epithelial tumors in a subject.

Diagnosis as used in the context of the present invention may comprise determining the level of TAP-70 molecules in a sample. Based upon the determined level of TAP-70 in the samples individuals can be subdivided into subgroups. The subgroups may be created according to clinical data, such as e.g. survival, recurrence of disease, frequency of metastases etc., related to the particular level of TAP-70 molecules determined in the samples. Based upon these subgroups for example an assessment of prognosis may be done. According to the subgroups the therapy of the individuals affected by the tumors may be tailored.

Monitoring may comprise detecting the level of TAP-70 in samples taken at different points in time and determining the changes in said level. According to said changes the course of the disease can be followed. The course of the disease may be used to select therapy strategies for the particular individual.

Another aspect of diagnosis and monitoring of the disease course according to the present invention may comprise the detection of minimal residual disease. This may comprise for example the detection of a TAP-70 level in one or more body samples following initial therapy of an individual once or at several timepoints. According to the level of TAP-70 detected in the samples one may select a suitable therapy for the particular individual. Generally the detection of the TAP-70 molecules in biological samples may comprise:

(a)determining the presence or absence and/or the amount of expression of the inventive TAP-70 polypeptide in a biological sample; and
(b) diagnosing an epithelial tumor or a susceptibility to an epithelial tumor based on the presence or amount of expression of the polypeptide.

The present invention further relates to a method for detecting a level of TAP-70 molecules in a biological sample comprising at least two of the following steps:

(a) contacting a biological sample obtained from a patient with a probe that is capable of binding to a nucleic acid molecule according to claim 1 or a polypeptide according to claim 6 or 8; and
(b) determining in the sample the presence or absence or an amount of nucleic acid molecules or polypeptides that bind to said probe.
(c) comparing the detected amount to a control amount corresponding to wild type conditions

Suitable approaches for carrying out the diagnostic method of the invention are described below as well as in the appended examples.
Biological sample as used herein may comprise any sample comprising cells or cell debris. Biological samples may comprise samples of clinical relevance, such as e.g. secretions, smears, body fluids, urine, semen, stool, bile, biopsies, cell- and tissue-samples. Biopsies as used in the context of the present invention may comprise e.g. resection samples of tumors, tissue samples prepared by endoscopic means or needle biopsies of organs. Furthermore any sample potentially containing the marker molecules to be detected may be a sample according to the present invention. Such samples may comprise for example intact cells, lysed cells or any liquids containing proteins, peptides or nucleic acids. Even solids, to which cells, cell fragments or marker molecules, such as TAP-70 nucleic acids or TAP-70 proteins, may adhere may be samples according to the present invention. Such solids may comprise for example membranes, glass slides, beads etc..Preparation of a sample may comprise e.g. obtaining a sample of a tissue, a body fluid, of cells, of cell debris from a patient. According to the present invention preparation of the sample may also comprise several steps of further preparations of the sample, such as preparation of dissections, preparation of lysed cells, preparation of tissue arrays, isolation of polypeptides or nucleic acids, preparation of solid phase fixed peptides or nucleic acids or preparation of beads, membranes or slides to which the molecules to be determined are coupled covalently or non-covalently.
The present invention also relates to a diagnostic composition containing a nucleic acid molecule, polypeptide and/or antibody of the invention. Said diagnostic composition can be in form of a kit. Such kits are useful for the detection of a target cellular component, which is TAP-70 or, alternatively, TAP-70 encoding mRNA, wherein an increased concentration of TAP-70 (compared to the concentration in normal tissue) or, alternatively, TAP-70 encoding mRNA is indicative for a epithelial tumor or a disposition for such a tumor.
The TAP-70 polypeptide or the corresponding mRNA, e.g. in biological fluids or tissues, may be detected directly in situ, e.g. by in situ hybridization (e.g., according to the examples, below) or it may be isolated from other cell components by common methods known to those skilled in the art before contacting with a probe. Detection methods include Northern Blot analysis, RNase protection, in situ methods, e.g. in situ hybridization, in vitro amplification methods (PCR, LCR, QRNA replicase or RNA-transcription/amplification (TAS, 3SR), reverse dot blot disclosed in EP-B1 0 237 362)), immunoassays, Western Blot and other detection assays that are known to those skilled in the art.
The probe (e.g. a specific -antibody or specific oligonucleotide) of the diagnostic composition (or kit) can be detectably labeled. In a preferred embodiment, said kit contains an anti-TAP-70 antibody and allows said diagnosis, e.g., by ELISA and contains the antibody bound to a solid support, for example, a polystyrene microtiter dish or nitrocellulose paper, using techniques known in the art. Alternatively, said kits are based on a RIA and contain said antibody marked with a radioactive isotope. In a preferred embodiment of the kit of the invention the antibody is labeled. Suitable antibody assay labels are known in the art and include enzyme labels, such as, glucose oxidase, and radioisotopes, such as iodine ($^{125}I$, $^{121}I$), carbon ($^{14}C$), sulfur ($^{35}S$), tritium ($^{3}H$), indium ($^{112}In$), and technetium rhodamine, and biotin. In addition to assaying TAP-70 levels in a biological sample, the polypeptide can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma. A protein-specific antibody or antibody fragment which has been labeled with an appropriate detectable imaging moiety, such as a radioisotope (for example, $^{131}I$, $^{112}In$, $^{99}mTc$), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of $^{99}mTc$. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments". (Chapter 13 in Tumor Imaging: The Radiochemical Detection of Cancer, S.W. Burchiel and B.A. Rhodes, eds., Masson Publishing Inc. (1982)).
The marker TAP-70 is also useful for prognosis, for monitoring the progression of an epithelial tumor and the diagnostic evaluation of the degree of malignancy of the tumor (grading and staging), e.g. by using in situ hybridization, e.g. according to the examples below.
The following examples illustrate the invention.

**Example 1**

**General methods**

**(A) Animals**

Female C57BL/6 mice aged 7-9 weeks and female NMRI mice (RCC, Füllinsdorf, Switzerland) as well as *c-fos*$^{-/-}$ mice (Wang et *al.,* Nature 360 (1992), 74-745were housed in specific pathogen free (SPF) and in light, temperature (21°C) and humidity (50%-60%) relative humidity) controlled conditions. Food and water were available *ad libitum.* The procedures for performing animal experiments were in accordance with the principles and guidelines of the ATBW (officials for animal welfare) and were approved by the Regierungsprasidium Karlsruhe.

**(B) Treatment of mouse skin**

C67BL/6, c-Fos$^{-/-}$ mice as well as their littermates were shaved on the dorsal skin and treated three days later topically with 10 nmol TPA (12-O-tetradecanoyl-13-phorbolacetate), which is the dose used for the promotion of skin tumors or with 10 nmol TPA plus 50 mg dexamethasone (Sigma, Chemical Co. St.Louis, USA) dissolved in 200 ml acetone. The animals were sacrificed 0-16 hours after TPA application. Hyperplastic skin was obtained from female NMRI mice. Three days before start of treatment seven weeks old animals were shaved, subsequently for a period of seven weeks single doses of 10 nmol TPA were dissolved in acetone and applied twice a week onto the dorsal skin. Four days after the last TPA application the animals were sacrificed and the skin was taken. Skin tumors derived from female NMRI mice used in this study were generated according to the initiation-promotion protocol of chemically induced multistage carcinogenesis (Fürstenberger and Kopp-Schneider, Carcinogenesis 16 (1995), 61-69). All tissues and tumors were isolated and immediately frozen in liquid nitrogen.

**(C) Isolation of poly(A)$^+$ RNA and cDNA synthesis**

Total RNA was isolated from human scalp, acetone-control and 6 h TPA treated mouse dorsal skin using RNeasy (AGS, Heidelberg, Germany) according to the manufacturer's recommendation. Poly(A)$^+$ RNA was purified using oligo(dT) coated Quiaex beats (Quiagen, Hilden, Germany). To synthesise double stranded cDNA aliquots of 2 mg poly(A)$^+$ RNA with 500 ng oligo (dT)-*RsaI* primer adapter (Clontech, Palo Alto, USA) in a volume of 11 ml were heated to 70°C for 10 min in a thermal cycler (Perkin Elmer 2400) and rapidly chilled on ice. The reaction mixture was made up to 20 ml by adding 4ml 5x first strand buffer (provided with the Superscript reverse transcriptase; Gibco BRL, Karlsruhe, Germany), 2 ml 0.1M DTT and 1 ml dNTP mix (10 mM each dATP, dGTP; dCTP and dTTP). Reverse transcription was started by adding 2 ml reverse transcriptase and incubated for 1 h. Subsequently, Klenow-mediated second strand cDNA synthesis was performed according to the instructions of the PCR-Select™ cDNA subtraction kit (Clontech, Palo Alto, USA).

**(D) Generation of a subtracted library using SSH**

SSH was performed between cDNA from acetone-control ("tester") and 6 h TPA treated mouse dorsal skin ("driver") using the PCR-Select™ cDNA subtraction kit (Clontech, Palo Alto, USA) according to the manufacturers recommendation, except for modifications of the PCR and hybridisation conditions. All PCR and hybridisation steps were performed on a Perkin Elmer 2400 thermal cycler. For the first hybridisation the mixture of "driver" and "tester" cDNAs was denatured at 100°C for 20 s and then cooled over 1 min to 68°C and maintained at this temperature for 8 h. For the second hybridisation, a two-fold excess of control "driver" cDNA was denatured at 100°C for 20 s and then added directly to the pooled mix of the two previous hybridisations and allowed to incubate at 68°C for 20 h. It was necessary to alter the PCR conditions (see below) such that the amplification of unaltered sequences was kept to a minimum.

**Used PCR-primer (Clontech)**

cDNA synthesis primer
5'-TTTTGTACAAGCTT$_{30}$N$_{1}$N-3'

Adaptor 1
5'-CTAATACGACTCACTATAGGGCTCGAGCGGCCGCCCGGGCAGGT-3'
..................................3'-GGCCCGTCCA-5'
PCR primer 1
5'-CTAATACGACTCACTATAGGGC-3'

Nested PCR primer 1
5'-TCGAGCGGCCGCCCGGGCAGGT-3'

Adaptor 2R
5'-CTAATACGACTCACTATAGGGCAGCGTGGTCGCGGCCGAGGT-3'
................................3'-GCCGGCTCCA-5'

Nested PCR primer 2R
5'-AGCGTGGTCGCGGCCGAGGT-3'

All other procedures for generation of the subtracted library were done according to the guidelines of the cDNA subtraction kit. PCR parameters were as follows: 20 cycles of 94°C for 20 s; 68°C for 30 s and 72°C for 2 min. The subtracted cDNA was subjected to a second round of PCR (nested), using the same PCR conditions with the exception that 14 cycles were performed. The subtracted cDNA library was cloned directly into the T/A vector pCRII. 1 (TA cloning kit, Invitrogen, De Schelp, Netherlands) and the ligation was transformed into Electromax™ bacterial strain DH10B (Life Science, Karlsruhe, Germany).

**(E) Generation of a human scalp cDNA library**

The human scalp cDNA was cloned into the vector pBS-SK and the ligation was transformed into Electromax bacterial strain DH10B (Life Science, Karlsruhe, Germany).

**(F) Reverse Northern high density blot analysis**

A total of 3,000 individual recombinant clones were picked and individually inocculated into sterile 96-well microtiter plates containing LB-medium and ampicillin at 100 mg/ml. After incubation of bacteria on a gyratory shaker for 8h at 37°C, equal amounts of the liquid cultures were spotted in duplicate onto nylon membranes (Hybond $N^+$ Amersham Pharmacia, Freiburg, Germany). The filters were hybridised under stringent conditions (7% SDS in 0.5 M $NaPO_4$, pH7.2; Church and Gilbert, 1984) at 65°C over night with equivalent amounts of $^{32}P$-labelled double stranded cDNA probes derived from acetone-control "driver" and 6 h TPA-treated murine dorsal skin "tester" mRNA respectively, which were prepared according to the recommendation of Roche Diagnostics' cDNA synthesis kit (Roche Diagnostics, Mannheim, Germany). Filters were washed under stringent conditions (see above) at 65°C. The filters were exposed to x-ray film up to 2 days at -80°C and the signals of corresponding clones were compared. The complex "driver" and "tester" cDNA probes were also used for Southern Blot analysis of *Eco*RI-digested plasmid of selected cDNA clones.

**(G) Northern blot analysis**

Total RNA was isolated from cell lines, from 6 h acetone-, TPA- as well as TPA plus Dexamethasone-treated murine skin and from squamous cell carcinomas, as described previously (Tuckermann et al., J.Cell.Biol. 147 (1999), 1365-1370). 15 mg total RNA were fractionated on 1.4% formaldehyde-agarose gels and subjected to Northern Blot analysis using an [a-$^{32}P$]dCTP-labelled murine TAP-70 probe (nucleotides 710-1948), which was isolated by EcoRI digestion of the appropriate pCR2.1-plasmid (DSM 14831 = plasmid 3'mTAP70) and a human TAP-70 probe (nucleotides 168-1695) isolated by EcoRI/Xho1 digestion of the pBS-SK plasmid (DSM 14830 = plasmid hcTAP70).. The probe for 18S rRNA was obtained by RT-PCR using RNA from mouse skin.

**(H) In Situ Hybridisation**

*In situ* hybridisation was performed on six µm paraffin sections as described in detail (Gack et al, Cell Growth Differ. 6 (1995), 759-767). All the samples were fixed in 4% paraformaldehyd and treated with proteinase K (0.3 mg/ml), subsequently they were washed in 0.1 mol/l triethanolamine buffer containing 0.25% acetic anhydride. The sections were covered with 20-100 ml of hybridisation buffer containing 1.5 x $10^6$ cpm of $^{35}$S-labeled antisense or sense RNA probe (see below), and incubated at 53°C for 18h in a humidified chamber. After hybridisation, the slides were washed under stringent conditions (50% formamid, 65°C), including treatment with RNAse A (20 mg/ml) to remove unhybridised probe. After 7-21 d of autoradiography, the photographic emulsion (NTB2; Kodak, München, Germany) was developed, and the slides were stained with hematoxylin and eosin. Each sample was hybridised in at least two experiments. The cRNA probes were derived by in *vitro* transcription for the human TAP-70 from a BamHI linearized recombinat plasmid pBS-SK (DSM 14830 = plasmid hcTAP70) containing a 1,528 bp fragment (nucleotides 168-1,695 of the sumitted sequence, see figure 1b) and for the murine TAP-70 from a BamHI linearized recombinat plasmid pCR2.1 (DSM 14831 = plasmid 3'mTAP70) containing a 1,239 bp fragment (nucleotides 710-1,948 of the submitted sequence, see figure 2b) As controls for nonspecific hybridisation, sections were hybridised with the appropriate sense probes.

**(I) Immunohistochemistry**

Six mm thick paraffin sections from skin biopsies were treated as described previously (Schnarr et al., Int.J. Cancer 89 (2000), 506-513) followed by incubation with anti-TAP-70 antibodies (10 mg/ml final concentration; generated by using the peptide shown in Figure 2 as immunogen (amino acid sequence: SRRMATSGVRSKEGRRE) with 50 mM Tris-buffer (pH 7.4) sections were incubated with 18 mg/ml goat anti-rabbit IgG (Dianova, Hamburg, Germany) followed by two cycles of incubation with an 1:10 diluted mouse alkaline phosphatase-anti-alkaline-phosphatase complex (APAAP, Linaris, München, Germany). After washing with distilled water for 5 min, sections were incubated with naphthol AS-BI phosphate (Sigma, München, Germany) as substrate and stained with fuchsin (Sigma, München, Germany) as chromogen. Blocking of the endogenous alkaline phosphatase was achieved by adding 1.73 mM levamisol (Sigma, München, Germany).

**Example 2**

**Isolation of TAP-70 encoding cDNAs**

A clone encoding the murine TAP-70 (DSM 14831 = plasmid 3'mTAP70) was isolated from the cDNA library described in Example 1 using the following primers (Clontech)

cDNA synthesis primer
5'-TTTTGTACAAGCTT$_{30}$N$_{1}$N-3'

Adaptor 1
5'-CTAATACGACTCACTATAGGGCTCGAGCGGCCGCCCGGGCAGGT-3'
..................................3'-GGCCCGTCCA-5'

PCR primer 1
5'-CTAATACGACTCACTATAGGGC-3'

Nested PCR primer 1
5'-TCGAGCGGCCGCCCGGGCAGGT-3'

Adaptor 2R
5'-CTAATACGACTCACTATAGGGCAGCGTGGTCGCGGCCGAGGT-3'
................................3'-GCCGGCTCCA-5'

Nested PCR primer 2R
5'-AGCGTGGTCGCGGCCGAGGT-3'

according to the method described in Breitenbach et al., J.Invest.Dermatol. 117 (2001), 634-640). The nucleotide sequence and the derived amino acid sequence are shown in Figure 2. The protein has a calculated MW of about 45,000 Da. The peptide used for generation of polyclonal antibodies is marked

The nucleotide sequence and derived amino acid sequence of the corresponding human TAP-70 cDNA are shown in Figure 1. This nucleotide sequence was obtained by screening a human scalp cDNA library and cloning the appropriate fragment which hybridized under low stringency with the [32P]dCTP-labelled murine TAP-70 probe (nucleotides 710-1948), which was isolated by EcoRI digestion of the appropriate pCR2.1-plasmid (DSM 14831 = plasmid 3'mTAP70).

**Example 3**

**TAP-70 expression in TPA-treated skin and in TAP-induced tumors**

The expression of TAP-70 in TPA-treated skin and in TPA-induced papillomas and carcinomas was studied by in situ-hybridization as described in Example 1, above, and Breitenbach et al., 2001. The results are presented in Figures 3 and 4. As shown in Figure 3A, TAP-70 is expressed in TPA-treated murine skin in keratinocytes of a late stage of differentiation. As regards papillomas and carcinomas, TAP-70 expression can be observed in tumor cells showing weak proliferation at a late stage of differentiation (Figures 3B and 3C). The results of Western Blot analyses using the anti-TAP-70 antibody described in Example 1, above, are shown in Figure 4A. A band corresponding to a cytosolic protein with a relative molecular weight of about 50,000 Da is clearly detectable in the TPA-treated PMKR3 celline (murine SV40-T-antigen immortalized keratinoyctes established by Petra A. Rehberger (1997) Expression von zellzyklusregulierten Proteinen im Zusamenhang mit Proliferation und prograrimiertem Zelltod in Keratinozyten, PhD. Thesis, Ruprecht-Karl-Universität, Heidelberg)

The results of immunohistochemical and of in situ-hybridization analyses show similar results as shown shown in Figures 4B and 4C. A high TAP-70 expression on the RNA and the protein level can be observed in chronic hyperplastic skin, papillomas and carcinomas, i.e. tumor cells having a low proliferation index (demonstrated by low PCNA staining) at a late stage of differentiation.

**Example 4**

**Expression of TAP-70 in human tumors (skin, colon)**

For the analyses of expression of TAP-70 in human skin tumors two different sources (a) and (b) were used. (a) were commercially available section samples of different kinds of tumors fixed to the surface of slides (Figure 5A);

and (b) sections of skin tumors showing a different degree of malignancy (Figure 5B; keratoacanthoma, carcinoma, basaloma). The results obtained with samples (a) and (b) are in principle identical: There is high expression of TAP-70 in tumor cells showing a late stage of differentiation.

Figure 5C shows the results of Northern Blot analyses. RNA was prepared from from the carcinoma shown in Figure 5B and hybridized with the probe described in Example 2. A band corresponding to an mRNA with a length of about 1.9 kb (corresponding to the length of the cDNA) is detectable.

Finally, the expression of the gene encoding TAP-70 was analysed in different colon carcinomas by use of real time PCR. The results are summarized in Figure 5D.

Samples of 15 colon carcinomas were used to determine the level of TAP 70 mRNA using semi-quantitative RT PCR. Colon carcinoma samples were collected, snap frozen, and stored at-80°C. They were verified to be composed predominantly of neoplastic cells by histopathological analysis. mRNA was isolated from tumors and patient-matched normal tissue using Qiagen reagents (Qiagen, Hilden, Germany), and single-stranded cDNA was synthesized using Superscript II (Life Technologies, Inc.). Quantitative PCR was performed using the 7700 Sequence Detector (TaqmanTM) and the SYBR Green PCR Master-Mix, as described in the manufacturers manual (Applied Biosystems, Foster City, CA).

PCR reactions were performed in 25 µl volumes with a final concentration of 300 nmol for each primer, with 95°C for 15 sec and 60°C for 60 sec, for 40 cycles. The following primers are used for quantitative PCR:

Primer A: GTC TTT GCC AAC AGC ATG G

Primer B: CAG AGT CCA CCA GGA ACC TC

The specificity of the PCR products was verified by gel electrophoresis (data not shown).

The comparison of the expression data for carcinomas were compared to the data for normal tissue. The results show, that TAP 70 was expressed significantly higher than in control tissue in 10 out of 15 samples. This indicates a clear overexpression of the TAP 70 gene in the tested tumor samples compared to the normal tissue.

**Example 5**

**TAP-70 contains the consensus sequence of the active site of a aspartyl proteinase**

The kind of protein and the active site of TAP-70 have been identified applying the sequence alignment program Fugue alignment, and the programs Prosite Search and Pfam7.0-domains based on databases of protein families and of motifs.

The amino acid sequence of the active site of TAP-70 (mouse/human) FLVDSGAQVSVV shows 100% identity with the consensus sequences of aspartyl proteinases. The remaining regions of TAP-70 do not show any striking homologies with the other aspartyl proteases, e.g. Cathepsin B and D.

## Claims

1. A nucleic acid molecule encoding the human epithelial tumor associated polypeptide Tap-70 or a polypeptide exhibiting a biological property of TAP-70 and being selected from the group consisting of

    (a) a nucleic acid molecule encoding a polypeptide that comprises the amino acid sequence as depicted in Figure 1 or 2;
    (b) a nucleic acid molecule comprising the nucleotide sequence as depicted in Figure 1 or 2;
    (c) a nucleic acid molecule included in DSMZ Deposit No.

    DSM 14829 (= plasmid 5'hTAP70)

    DSM 14830 (= plasmid hcTAP70)

    DSM 14831 (= plasmid 3'mTAP70)

    DSM 14832 (= plasmid mgcTAP70)

    (d) a nucleic acid molecule encoding a polypeptide the sequence of which shows at least 40% identity to the amino acid sequence of the polypeptide encoded by a nucleic acid molecule specified in (a) to (c);
    (e) a nucleic acid molecule the sequence of which differs from the sequence of a nucleic acid molecule of (a) to (d) due to the degeneracy of the genetic code; and
    (f) a nucleic acid molecule, which represents a fragment or an allelic variant of a nucleic acid molecule of (a) to (e).
    (g) a nucleic acid, which encodes a fragment or a variant of the amino acid sequence depicted in Figure 2, which has an increased or decreased biological activity compared to the wild type amino acid sequence.

2. A recombinant vector containing the nucleic acid molecule of claim 1

3. The recombinant vector of claim 2 wherein the nucleic acid molecule is operatively linked to regulatory elements allowing transcription and synthesis of a translatable RNA in prokaryotic and/or eukaryotic host cells.

4. A recombinant host cell which contains the recombinant vector of claim 2 or 3.

5. The recombinant host cell of claim 4, which is a mammalian cell, a bacterial cell, an insect cell or a yeast cell.

6. A isolated epithelial tumor associated TAP-70 polypeptide or a polypeptide exhibiting a biological property of the human epithelial tumor associated polypeptide TAP-70 being selected from a group consisting of

    (f) a polypeptide, which is encoded by a nucleic acid molecule of claim 1.
    (g) a polypeptide, which comprises an amino acid sequence given in Figure 1 or 2;
    (h) a polypeptide, that is recognized by a binding agent, that has been raised against and is specifically binding the polypeptide of (a) or (b);
    (i) a fragment or a variant of the polypeptides of (a)-(c), that is encoded by a nucleic acid sequence, that hybridizes to a nucleic acid according to claim 1 under stringent conditions; and
    (j) a fragment or variant of the polypeptides of (a)-(d), which has an increased or decreased biological activity compared to the wild type TAP-70 polypeptide.

7. A method of making a polypeptide exhibiting a biological property of the human epithelial associated polypeptide Tap-70 comprising:

    (a) culturing the recombinant host cell of claim 4 and 5 under conditions such that said polypeptide is expressed; and
    (b) recovering said polypeptide

**8.** A polypeptide produced by the method of claim 7.

**9.** A fusion polypeptide comprising the polypeptide of claim 6 and/or 8.

**10.** An antisense RNA sequence **characterized in that** it is complementary or reverse-complementary to an mRNA transcribed from a nucleic acid molecule of claim 1 or a part thereof and can selectively bind to said mRNA or part thereof, said sequence being capable of inhibiting the synthesis of the polypeptide encoded by said nucleic acid molecule.

**11.** A ribozyme **characterized in that** it is complementary or reverse-complementary to an mRNA transcribed from a nucleic acid molecule of claim 1 or a part thereof and can selectively bind to and cleave said mRNA or part thereof, thus inhibiting the synthesis of the protein encoded by said nucleic acid molecule.

**12.** A binding agent directed against and specifically recognizing the polypeptide of claim 6 or 8 selected from the following

(a) an antibody;
(b) a fragment of an antibody;
(c) a peptidomimetic compound comprising an immunogen binding epitope;
(d) an oligopeptide capable of specifically binding to antigens;.

**13.** The nucleic acid molecule of claim 1, the polypeptide of claim 6 or 8, or the binding agent of claim 12 which is detectably labeled.

**14.** The nucleic acid molecule, the polypeptide or the binding agent of claim 12, wherein the label is selected from the group consisting of a radioisotope, a bioluminescent compound, a chemiluminescent compound, a fluorescent compound, a metal chelate, or an enzyme.

**15.** A transgenic non-human animal comprising at least one polynucleotide of claim 1 or the recombinant vector of claim 2 or 3.

**16.** The transgenic non-human animal of claim 15 further comprising at least one inactivated wild type allele of the corresponding TAP-70 encoding gene.

**17.** The transgenic non-human animal of claim 15 or 16 which is a mouse or rat.

**18.** A method for identifying a binding partner to a polypeptide of claim 6 or 8 comprising:

(a) contacting a polypeptide of claim 6 or 8 with a compound to be screened; and
(b) determining whether the compound effects an activity of said polypeptide or whether binding of the compound to said polypeptide has occured.

**19.** A method for identifying activators/agonists or inhibitors/antagonists of the TAP-70 polypeptide comprising the steps of:

(a) incubating a candidate compound with a polypeptide of claim 6 or 8;
(b) assaying a biological activity, and
(c) determining if a biological activity of said polypeptide has been altered.

**20.** A method for identifying activators or inhibitors of the expression of TAP-70 polypeptide comprising the steps of:

(a) incubating a candidate compound with an in-vivo or in-vitro test system for protein expression or administering a compound to a test organism,
(b) detecting the level of the polypeptide of claim 6 or 8 within the test system or within the organism, and
(c) determining if the level of said polypeptide has been altered.

**21.** A method of identifying and obtaining a drug candidate for therapy of a epithelial tumor comprising the steps of

(a) contacting the polypeptide of claim 6 or 8 or a cell expressing said polypeptide in the presence of components capable of providing a detectable signal in response to protein degradation, cell proliferation or cell differentiation with said drug candidate to be screened under conditions to allow protein degradation, cell proliferation or changes in cell differentiation and
(b) detecting presence or absence of a signal or increase of the signal generated from protein degradation, cell proliferation or cell differentiation, wherein the presence or increase of the signal is indicative for a putative drug.

**22.** The method of claim 21 wherein said cell is comprised in the transgenic non-human animal of one of the claims 16 or 17.

**23.** An activator/agonist or inhibitor/antagonist of the polypeptide of claim 6 or 8, an activator or inhibitor of the expression of the polypeptide of claims 6 or 8 or a binding partner of the polypeptide of claim 6 or 8 obtainable by one of the methods of claims 18 to 20.

**24.** A pharmaceutical composition comprising, a polynucleotide of claim 1, a polypeptide of claim 6, 8 or 9, a recombinant vector of any one of claims 2 to 4, an antisense RNA of claim 10, a ribozyme of claim 11, a binding agent of claim 12 or an activator/agonist, inhibitor/antagonist or binding partner of claim 23 and a pharmaceutically acceptable excipient, diluent or carrier.

**25.** Use of a polypeptide of claim 6 or 8, a recombinant vector of any one of claims 2 to 4, an antisense RNA of claim 10, a ribozyme of claim 11, a binding agent of claim 12 or an activator/agonist, inhibitor/antagonist or binding partner of claim 21 for the preparation of a medicament for treatment of disorders associated- with a non wild-type expression of the - TAP-70 molecules.

**26.** Use according to claim 25, wherein the disorder is an epithelial tumor.

**27.** A method for detecting a level of TAP-70 molecules in a biological sample comprising at least two of the following steps:

(a) contacting a biological sample obtained from a patient with a probe that is capable of binding to a nucleic acid molecule according to claim 1 or a polypeptide according to claim 6 or 8; and
(d) determining in the sample the presence or absence or an amount of nucleic acid molecules or polypeptides that bind to said probe.
(e) comparing the detected amount to a control amount corresponding to wild type conditions

**28.** The method of claim 27, which is used for the diagnosis, prognosis or monitoring of a disorder associated with a non wild type level of expression of the novel TAP-70 molecules.

**29.** The method of claim 28, wherein the disorder is an epithelial tumor or a susceptibility to an epithelial tumor in a subject.

**30.** The method of claim 27 or 28, which is used for diagnosis of a minimal residual disease in an individual.

**31.** A diagnostic composition containing a nucleic acid molecule of claim 1, a polypeptide of claim 6 or 8 and/or a binding agent of claim 10.

**32.** A research kit or diagnostic kit useful for the detection of a level of the novel TAP-70 molecules in a sample, said kit comprising at least one oligonucleotide probe and/or a binding agent of claim 12 capable of specifically binding to the nucleic acid molecule of claim 1 or the polypeptide of claim 6 or 8.

**33.** A research kit or diagnostic kit according to claim 32 for the detection of an epithelial tumor or a susceptibility to an epithelial tumor in a subject.

**34.** A diagnostic method according to any one of the claims 27 to 29, use according to claim 25 or 26 or a kit according to claims 31 or 32, for the detection of gastrointestinal or anogenital tumors.

```
hum70 cDNA
translated from  101 to 1132

  1     MGSPGASLGI KKALQSEQAT ALPASAPAVS QPTAPAPSCL PKAGQVIPTL
 51     LREAPFSSVI APTLLCGFLF LAWVAAEVPE ESSRMAGSGA RSEEGRRQHA
101     FVPEPFDGAN VVPNLWLHSF EVINDLNHWD HITKLRFLKE SLRGEALGVY
151     NRLSPQDQGD YGTVKEALLK AFGVPGAAPS HLPKEIVFAN SMGKGYYLKG
201     KIGKVPVRFL VDSGAQVSVV HPNLWEEVTD GDLDTLQPFE NVVKVANGAE
251     MKILGVWDTA VSLGKLKLKA QFLVANASAE EAIIGTDVLQ DHNAILDFEH
301     RTCTLKGKKF RLLPVGGSLE DEFDLELIEE DPSSEEGRQE LSH•
```

Fig. 1a

humane TAP-70 cDNA

```
1     CAAGGATGGA TGCAGAGGGT GAGCACCCAT CCTGCTAGTC CGGCCGGATG
51    CTGGCAGGAG GGCGGGGTGA GGAGGGGCGG AGCTTCCAGA ACAAAGGAGA
101   ATGGGGAGCC CAGGGGCCAG CCTAGGCATC AAAAAGGCTC TGCAGAGTGA
151   ACAGGCCACA GCACTGCCTG CCTCTGCCCC AGCAGTCAGC CAGCCGACCG
201   CGCCTGCTCC CTCCTGCTTG CCCAAGGCCG GGCAAGTCAT CCCCACTCTG
251   CTTCGAGAGG CCCCGTTTTC CAGCGTGATT GCGCCGACAC TGCTCTGTGG
301   GTTTCTCTTC TTGGCGTGGG TTGCTGCTGA GGTTCCAGAG GAGAGCAGCA
351   GGATGGCCGG GAGCGGAGCC AGGAGTGAGG AAGGCCGCCG GCAGCATGCC
401   TTCGTCCCGG AACCTTTTGA TGGGGCCAAT GTCGTCCCAA ACCTCTGGCT
451   GCACAGCTTT GAAGTCATCA ATGACCTCAA CCATTGGGAC CATATCACCA
501   AGCTAAGGTT CCTGAAAGAG TCCCTCAGAG GAGAGGCCCT GGGTGTCTAC
551   AATAGGCTCA GTCCCCAGGA CCAGGGAGAC TATGGGACTG TGAAAGAGGC
601   CCTCCTGAAG GCCTTTGGGG TCCCTGGGGC TGCCCCCAGC CACCTGCCCA
651   AAGAGATCGT CTTTGCCAAC AGCATGGGTA AGGGCTACTA TCTCAAGGGG
701   AAGATTGGCA AAGTGCCCGT GAGGTTCCTG GTGGACTCTG GGGCCCAGGT
751   CTCTGTGGTC CACCCAAACT TGTGGGAGGA GGTCACTGAT GGCGATCTGG
801   ACACCCTGCA GCCCTTTGAG AATGTGGTAA AGGTGGCCAA TGGTGCTGAA
851   ATGAAGATCC TGGGTGTCTG GGATACAGCG GTGTCCCTAG GCAAGCTGAA
901   GCTGAAGGCA CAGTTCCTAG TGGCCAATGC GAGTGCCGAG GAAGCCATCA
951   TTGGCACTGA TGTGCTCCAG GACCACAATG CTATCCTGGA CTTTGAGCAC
1001  CGCACATGCA CCCTGAAAGG GAAGAAGTTT CGCCTTCTGC CTGTGGGAGG
1051  GTCCCTGGAA GATGAGTTTG ACCTGGAGCT CATAGAGGAG GACCCCTCCT
1101  CAGAAGAAGG GCGGCAGGAG CTATCCCACT GAGAAGCCAC CTTTTCTTTA
1151  ACCTCCTAAA TATTGGTGGG AAGACCCACC GCTGTGGGGG GGGTTGCATA
1201  TCCTCATGGG GGTCACTGGG CTTGGCCAGT CTGCTTATCA ACTCTTGCTC
1251  TTCTCTCCCC TTTGCCTCCC TCTGCAGGGG CCTTAATCTG CCCCTGGTAG
1301  GGGAGGCTTC CACTGAACAG GCACAGGTGA GGGAGAGCAG GCTGGCTTAG
1351  AGGGACAGGG TCCCCATGGT CATCAAGCTG CTGTTGATGA CAAAGACTCA
1401  AAGGCTGGAA GAGCTCCCAA GGAAGCTAGA AATGCTTGTC TTTGAAAGAA
1451  CTGTGGGACC CCTTCAGATT CCCTGAGGTA TGGCTTGGTC ACTCTCAGGT
1501  CCTCAAAGCC TGTCTTAGTT GGGCTGGGTC CTAGCTGCAG GGTCTTTGTG
1551  AGGGTCACAG TTGCTCTGGG ACACCTCCCT GAAGAGCCTT TCCACCTGTA
1601  CAATCGTATT TTCTTTCTGT CATTTGCTTT GAAGCCCATT GTGCCTTATG
1651  CCAATAATTC AATTGCTGCA AACACCAATA AAGATTGATT CATGG
```

Fig. 1b

**murine TAP-70 protein**

murine TAP70-sequence translated from 179 to 1408

antigenic peptide underlined

MSSHLYPHLG YSRARLGRVP RLHPLTRAVL LTGWAGRCPV GTEGEAPILL
VRQDAGRRAG LGVELLEQRR MRNPGGPGWA SKRPLQKKQN TACLCAQQPA
RHFVPAPFNS SRQGKNTAQP TEPSLSSVIA PTLFCAFLYL ACVTAELPEV
SRRMATSGVR SKEGRREHAF VPEPFTGTNL APSLWLHRFE VIDDLNHWDH
ATKLRFLKES LKGDALDVYN GLSSQAQGDF SFVKQALLRA FGAPGEAFSE
PEEILFANSM GKGYYLKGKV GHVPVRFLVD SGAQVSVVHP ALWEEVTDGD
LDTLRPFNNV VKVANGAEMK ILGVWDTEIS LGKTKLKAEF LVANASAEEA
IIGTDVLQDH NAVLDFEHRT CTLKGKKFRL LPVGSSLEDE FDLELIEEEE
GSSAPEGSH•

Fig. 2a

```
1     TCCGCAGTTA ATTTAGAATG TATGAGTCAC CTTATCAAGG CAGGCTGTGA
51    GAGATGAGTG ACTGCAGATG CCTTCTCTTT GCCCCAAGCA GTGATGGGGT
101   GAGGCCAAAG GGGTCCCCTC TTCTGAAACA GGTAGAGACC TGCTTTCTGT
151   CTCCTCTTCT CTAATGATAA ACATCTGAAT GTCATCACAC CTGTATCCTC
201   ATCTGGGCTA TTCTAGGGCT AGGCTGGGGA GGGTCCCGAG GCTCCATCCC
251   CTGACCCGGG CTGTCTTACT CACTGGGTGG GCTGGCAGGT GTCCCGTAGG
301   TACTGAGGGT GAGGCACCAA TCCTGCTAGT CAGGCAAGAT GCTGGCAGGA
351   GGGCGGGGCT AGGGGTGGAG CTTCTAGAAC AAAGGAGAAT GAGGAACCCT
401   GGGGGCCCAG GTTGGGCATC AAAAAGGCCC CTGCAGAAGA AGCAGAACAC
451   AGCCTGCCTC TGTGCCCAGC AGCCAGCCAG ACACTTTGTA CCGGCTCCCT
501   TCAACTCGTC CAGGCAGGGC AAGAACACGG CCCAGCCGAC AGAGCCCTCG
551   CTCTCCAGCG TGATTGCGCC CACACTCTTC TGTGCGTTTC TTTACTTGGC
601   TTGTGTTACT GCTGAACTTC CAGAGGTGAG CAGAAGGATG GCCACCAGCG
651   GAGTCAGAAG CAAGGAAGGA CGCCGGGAGC ATGCCTTCGT CCCAGAACCT
701   TTCACTGGTA CTAACTTAGC TCCCAGCCTT TGGCTGCACC GCTTTGAAGT
751   CATTGATGAC CTCAACCATT GGGATCATGC CACCAAACTG AGGTTCCTGA
801   AAGAGTCGCT CAAGGGAGAT GCCCTGGATG TCTACAATGG ACTCAGTTCC
851   CAGGCCCAGG GCGATTTCAG TTTTGTGAAG CAAGCCCTCC TGAGGGCCTT
901   TGGGGCCCCT GGGGAGGCCT TCAGTGAGCC CGAAGAGATT TTGTTTGCCA
951   ACAGCATGGG TAAGGGCTAC TACCTTAAAG GGAAGGTTGG CCATGTGCCT
1001  GTGAGATTCC TGGTGGACTC TGGGGCTCAG GTGTCTGTGG TTCACCCCGC
1051  CTTATGGGAG GAGGTCACTG ATGGTGACCT GGATACTCTT CGTCCTTTTA
1101  ACAATGTGGT CAAAGTGGCC AATGGGGCAG AGATGAAGAT CTTGGGTGTG
1151  TGGGACACAG AAATTAGCCT GGGCAAGACA AAGCTGAAGG CCGAGTTTCT
1201  GGTGGCCAAC GCCAGCGCAG AAGAGGCTAT TATTGGCACA GACGTCTTGC
1251  AGGACCACAA TGCCGTGCTG GACTTCGAAC ACCGCACCTG CACCCTGAAG
1301  GGGAAGAAGT TCCGCCTGCT CCCTGTCGGG AGCTCCTTGG AGGATGAGTT
1351  TGACCTGGAG CTTATTGAGG AAGAGGAGGG GTCTTCTGCA CCGGAGGGCT
1401  CCCACTAAGA AACCCCATTT CTTGTTCCCA GCATTGGTAG GGGGACTTTG
1451  TGTTGGGGGG AGCAGATGTC CTGGGGGGTA TCATCCGGCC TAGCCAGTCT
1501  TTACACCGGT TCTCAGTTTC CCTCCTTCTA CAGGGGCCTT GCTTTGCCTT
1551  TGTTTGGGGA GGGAGGCCAG CTTGGTGGCC TAAAGCAGTG TCCCCAAGGT
1601  CTGCAAAGAC TTCCAAGGCT GGCAGGAGCT TCTGAGGAAG CCAGGAATGT
1651  CAATCTTGAG AGAGGACCCT TTTAGATCCC CTGAAGTATG GCTCAGTCAC
1701  TTTCACGTCC CCAAGCCTGC TGAGCTGAGC CTGGTCTTGG CTAAGACCCT
1751  CACAATCCAG ATGCTTGGAG GAGACTGGCA GCTGCTCTGG GAGTCCTCCC
1801  TGAGTCCTCC CACCTGCACA AGGATGCTCC CTCCTGTCCT GTCACTTGCC
1851  TTGAATCTCA TGGAGCCTGT ATCAATAATT CAATTATTTC AAAACACCAA
1901  TAAAGATCTG TTCATGTAAA AAAAAAAAAA AAAAAAAAAA AAAAAAAA
```

# Sequence Comparison #70 active center

murine HVPVRFLVDSGAQVSVVHP

| | | | | | | | | | | | | | | | | | | |

human KVPVRFLVDSGAQVSVVHP

Fig. 2b

# #70 in mouse skin

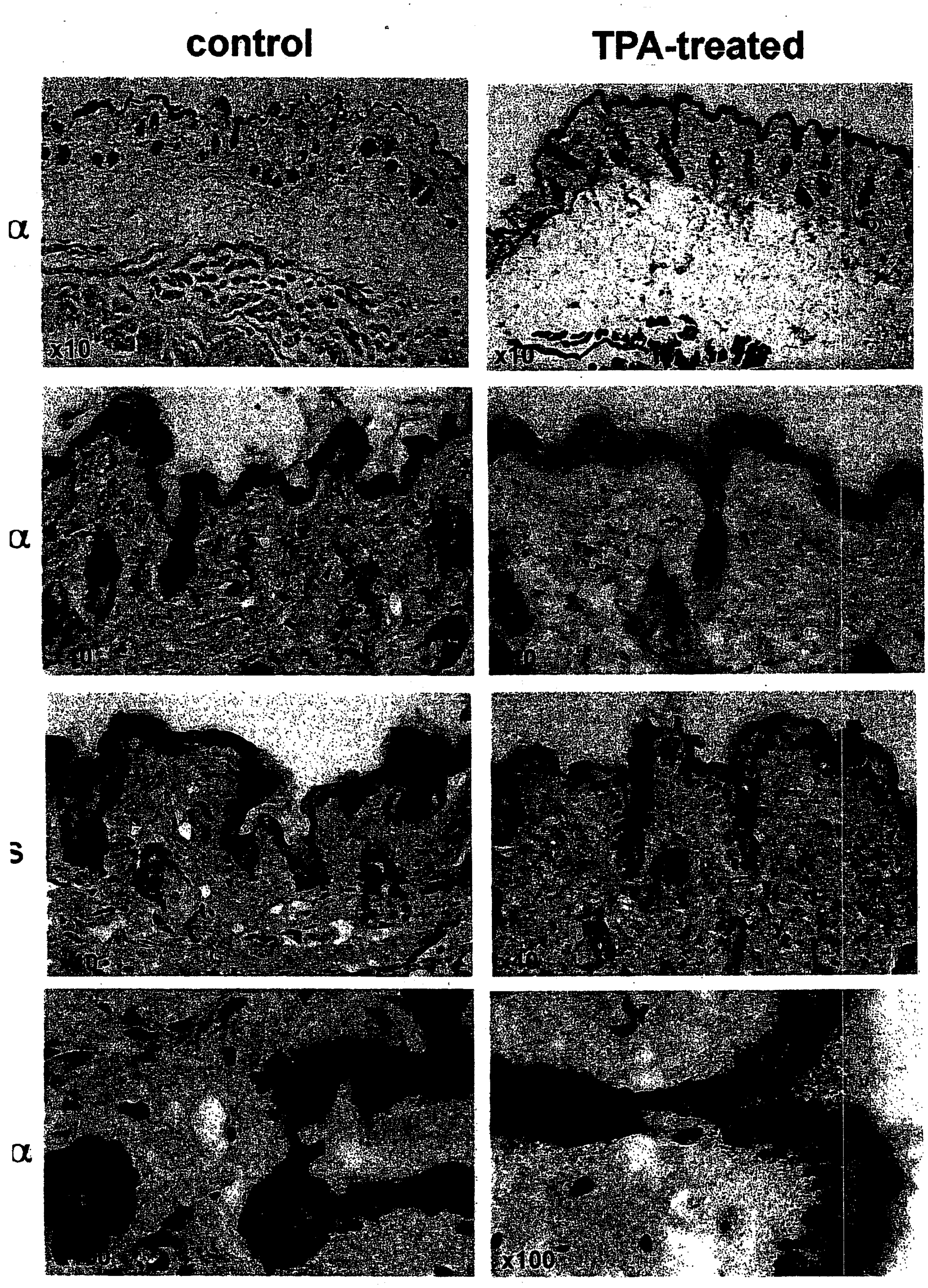


Fig. 3a

# #70 in papilloma

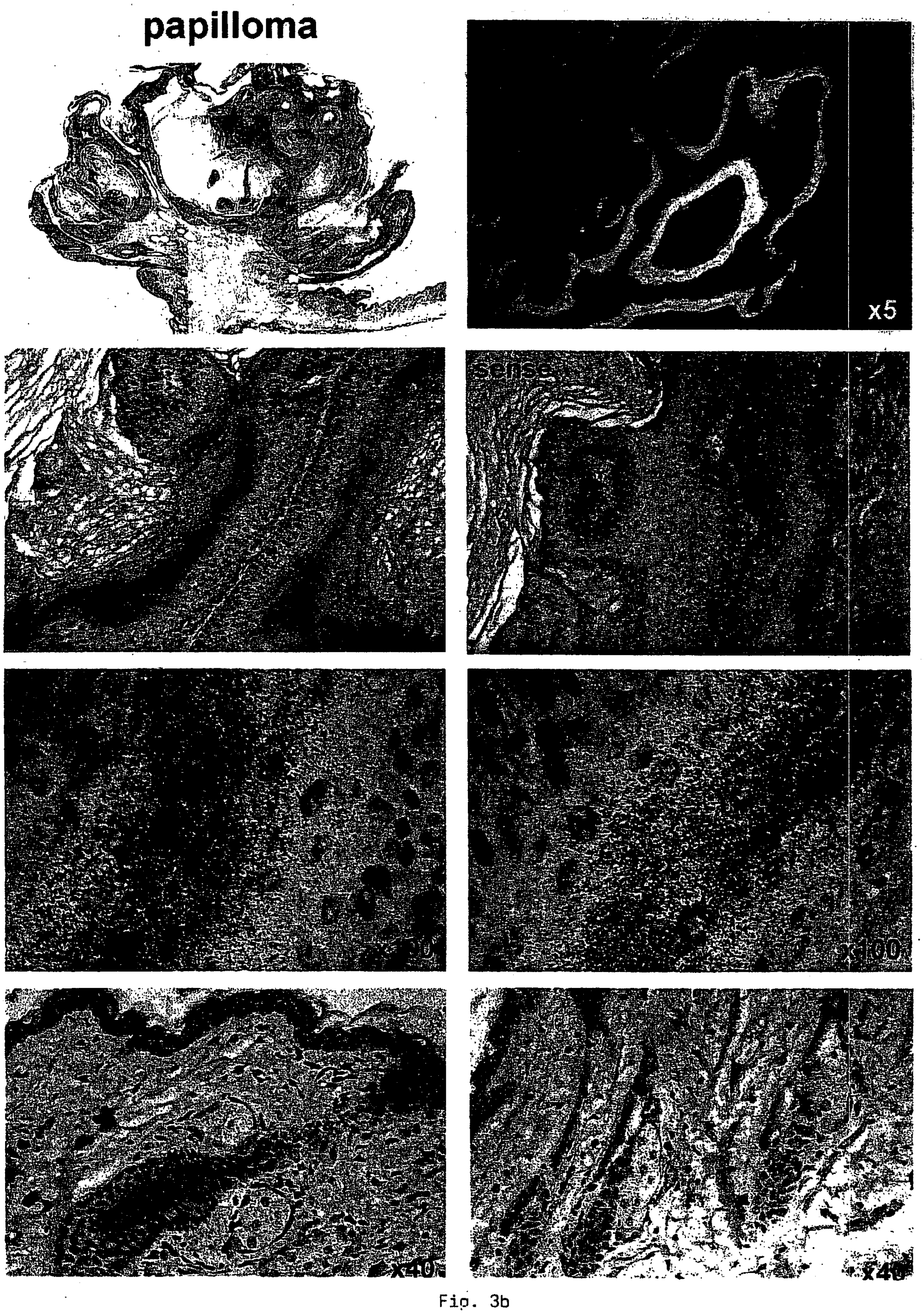


Fig. 3b

# #70 in squamous cell carcinoma

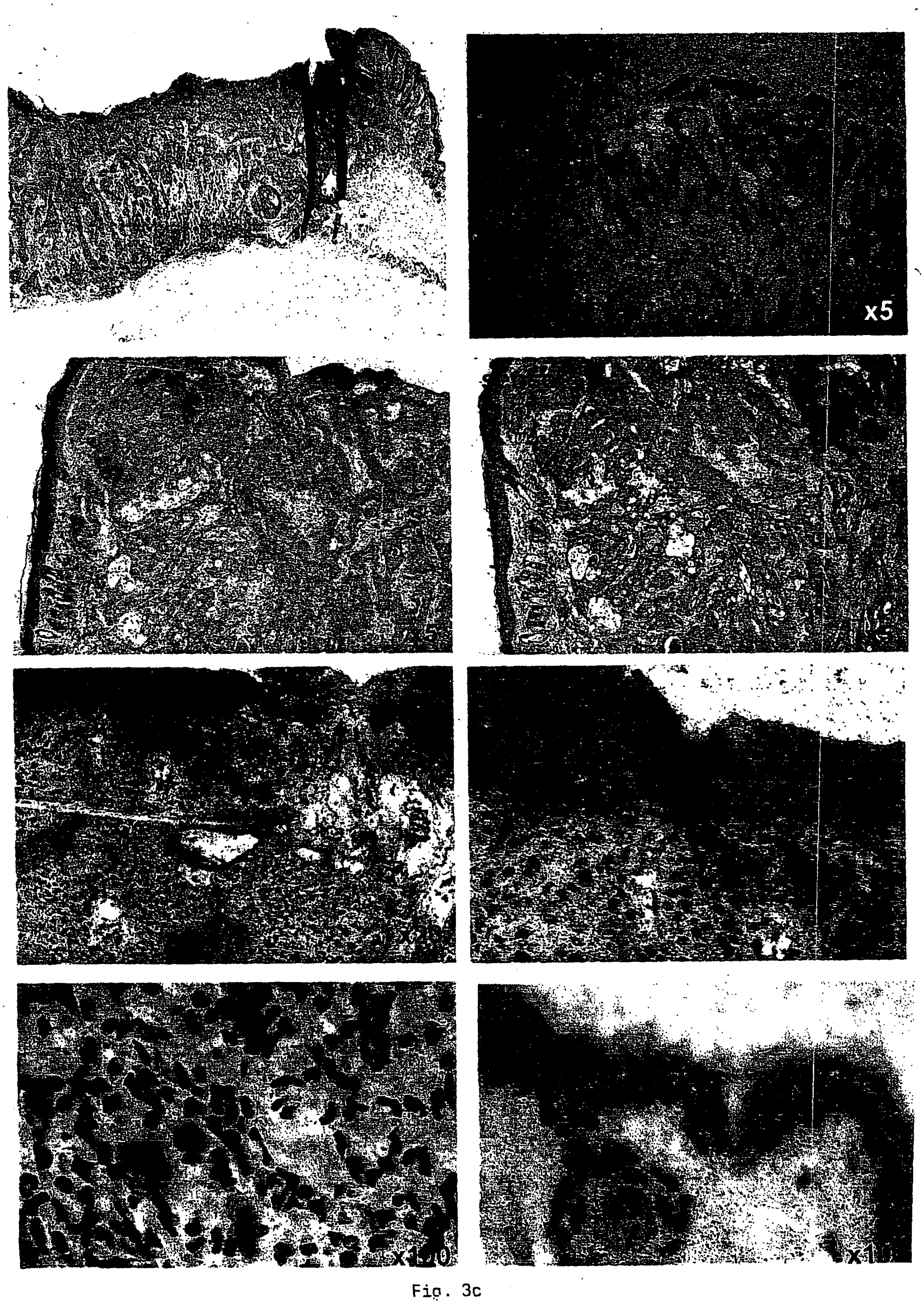


Fig. 3c

# Expression of 70 protein
# in primary mouse keratinocyte cell line PMK R3

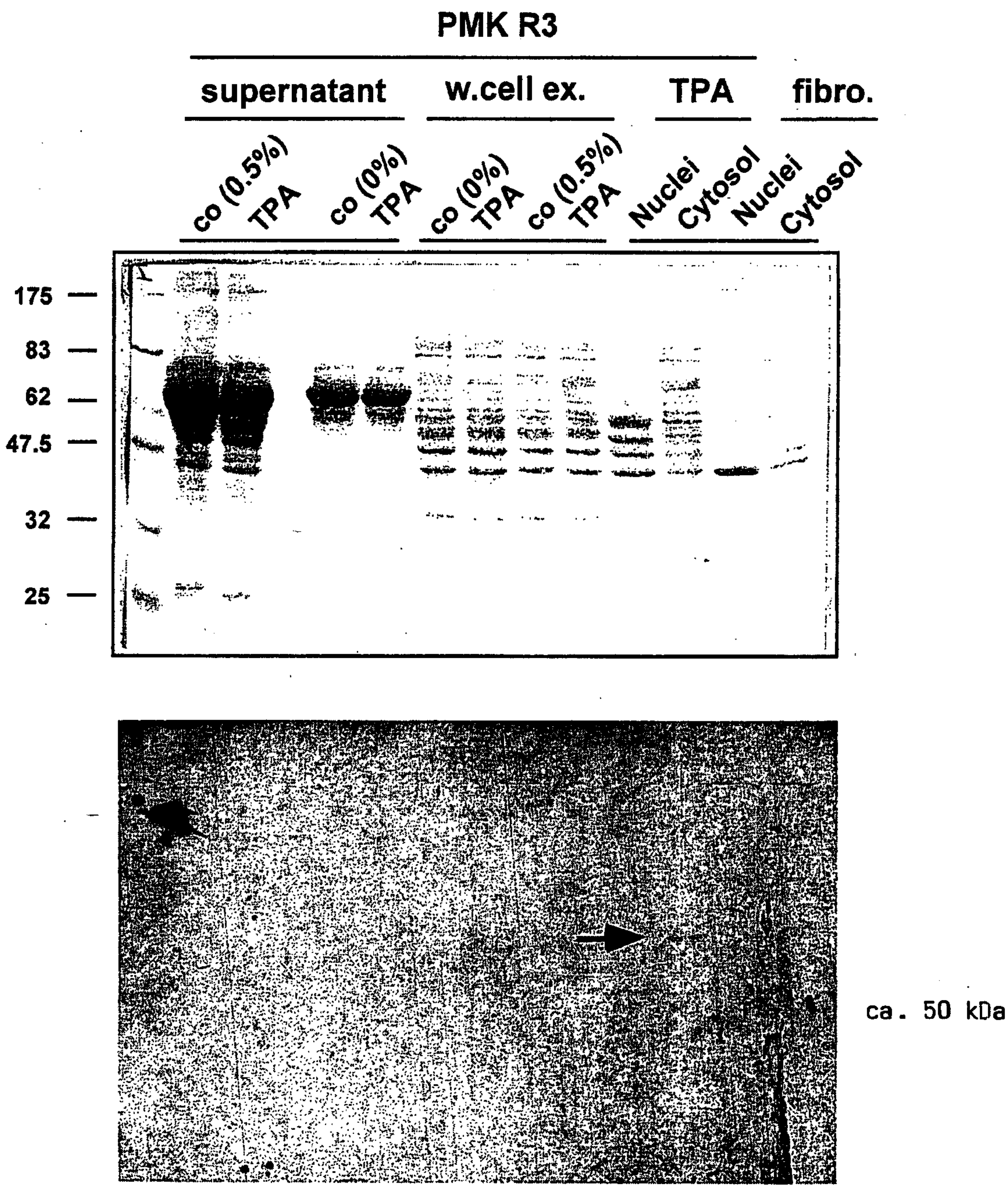


Fig. 4a

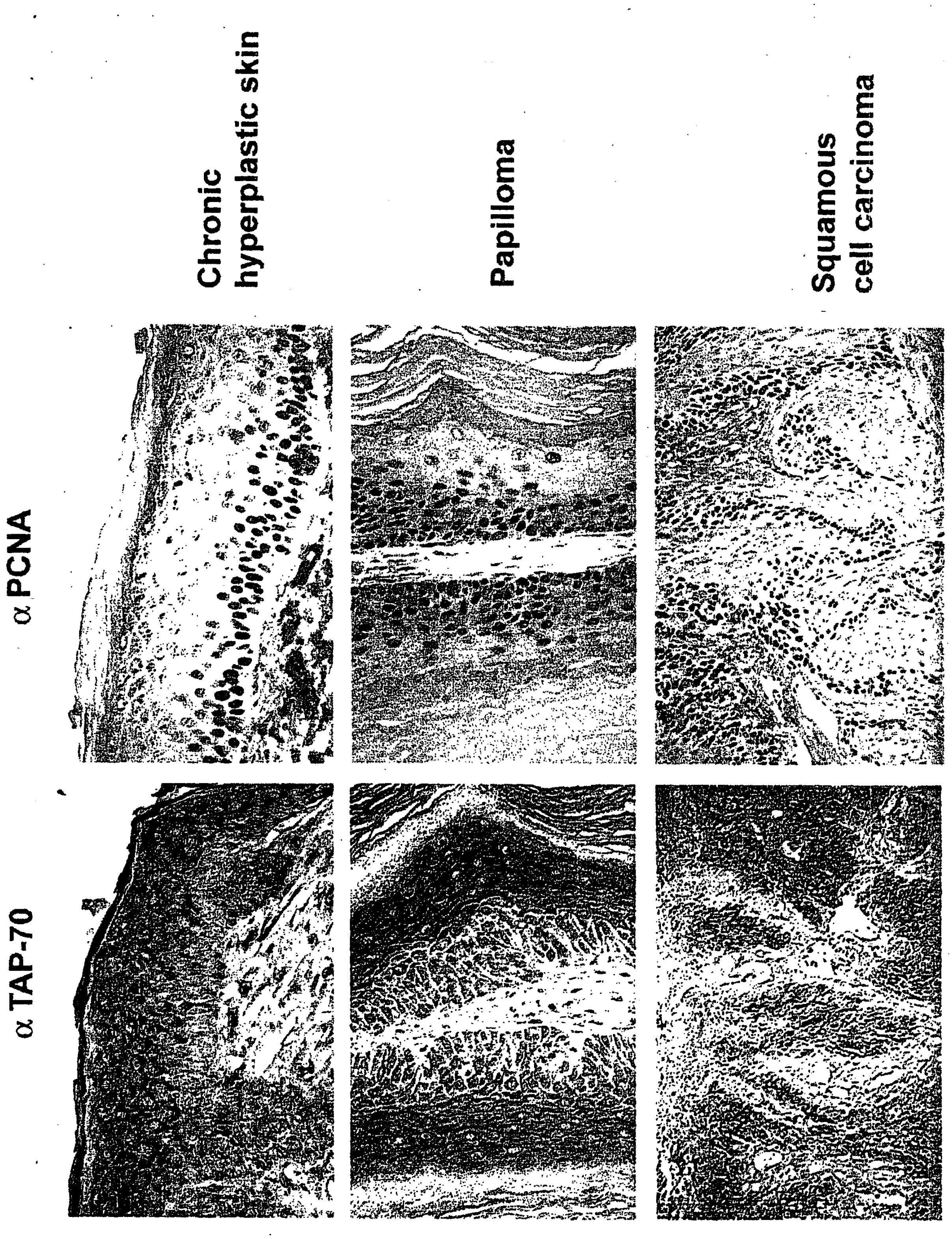
α PCNA
α TAP-70
Chronic hyperplastic skin
Papilloma
Squamous cell carcinoma

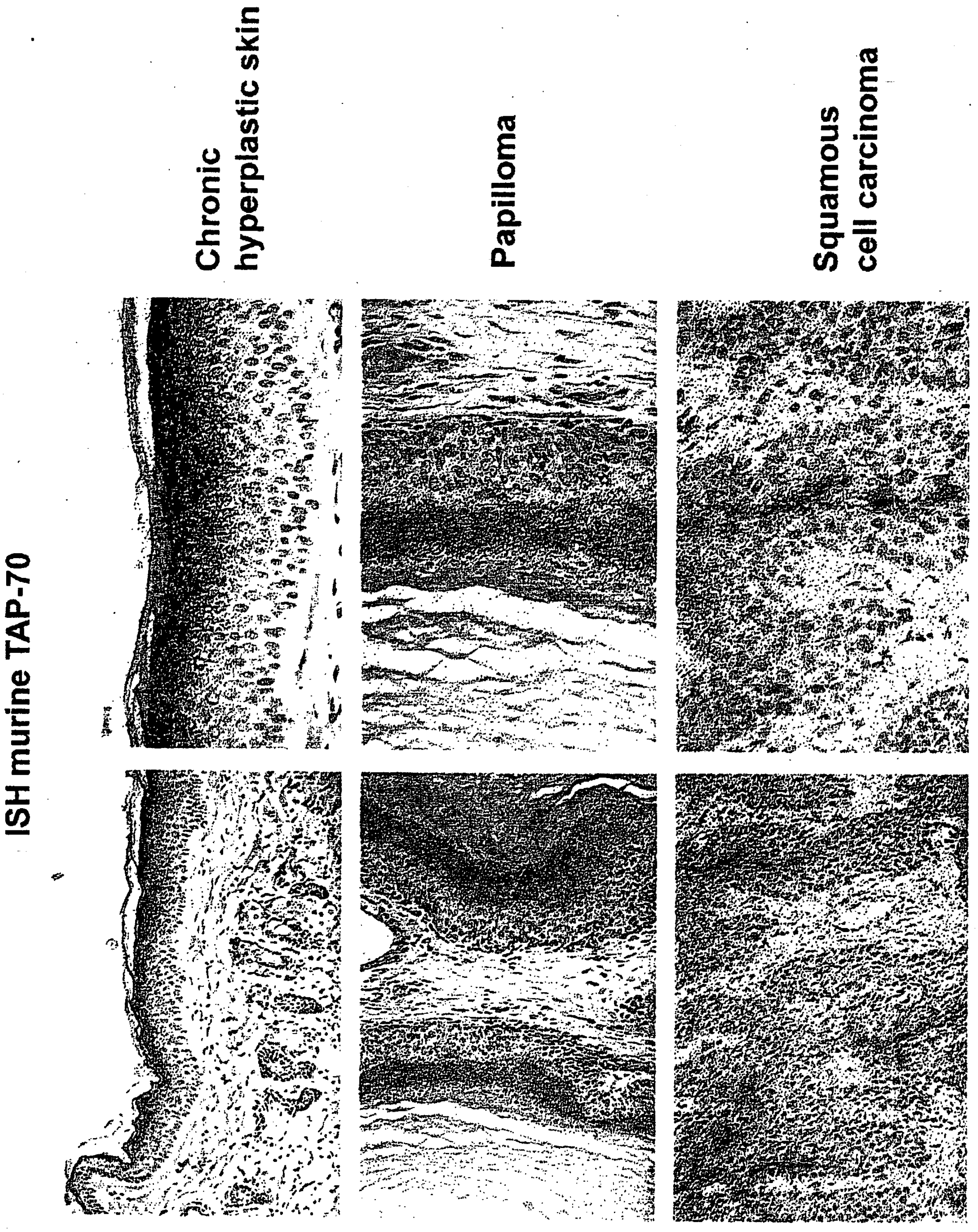
ISH murine TAP-70
Chronic hyperplastic skin
Papilloma
Squamous cell carcinoma

Fig. 4c

expression of TAP-70 in human skin carcinoma
detected by in situ hybridization ( BB3 )

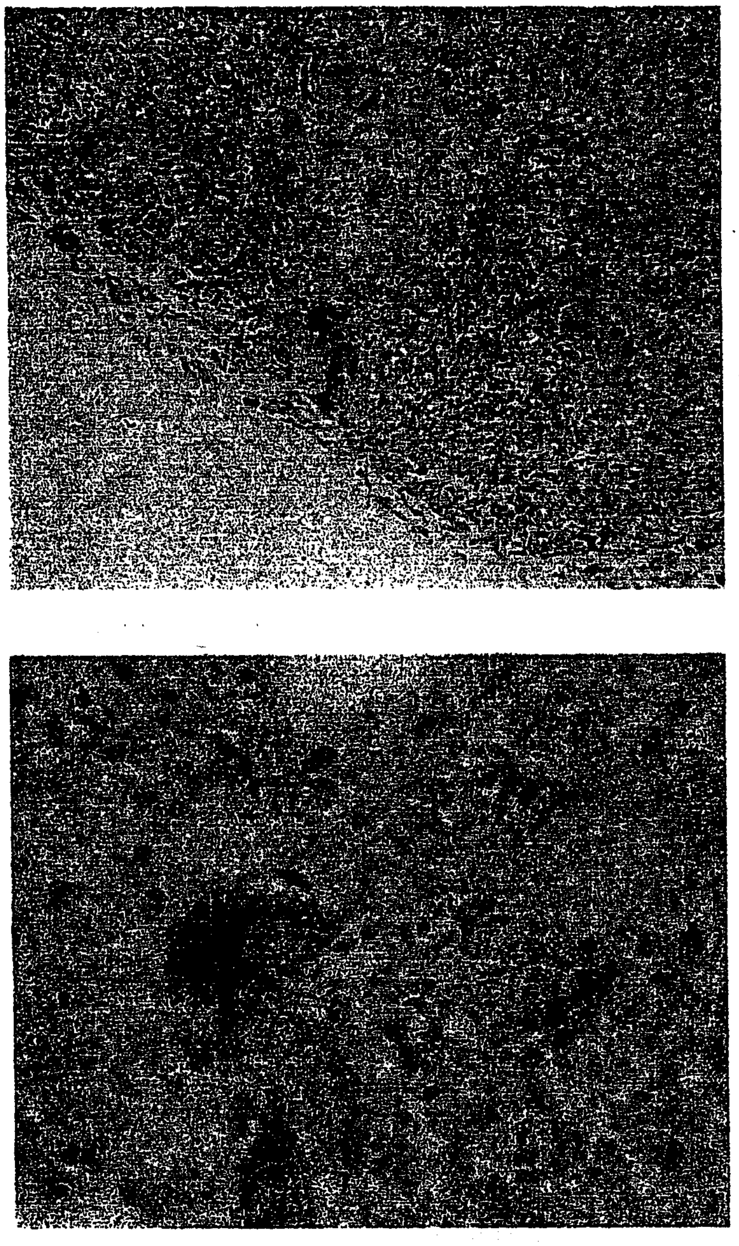

10x

40x

Fig. 5a

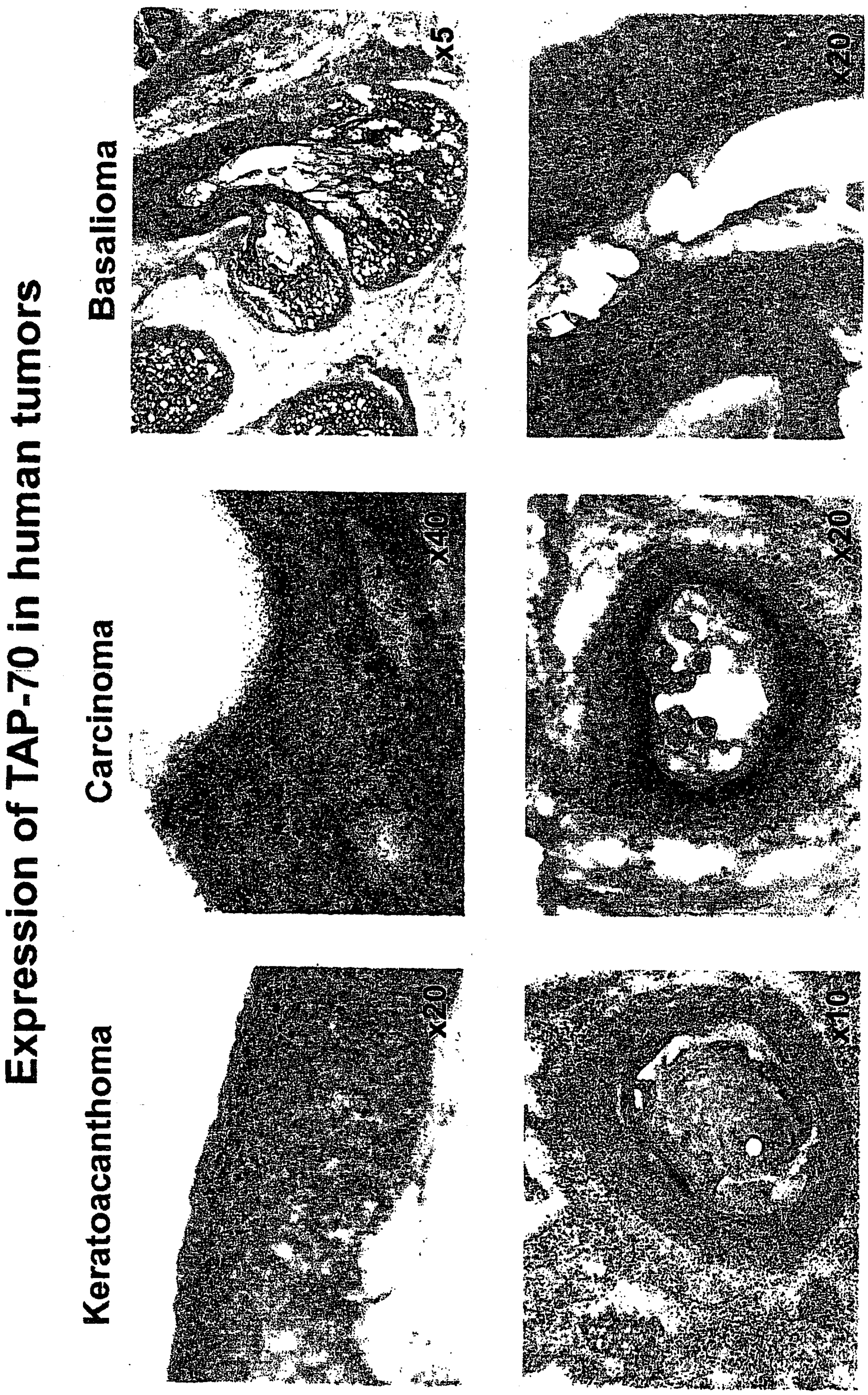
Expression of TAP-70 in human tumors
Keratoacanthoma
Carcinoma
Basalioma
x20
x40
x5
x10
x20
x20

Fig. 5b

**Squamous Cell Carcinoma**
**Nothern Blot Analysis**
**Human TAP-70**

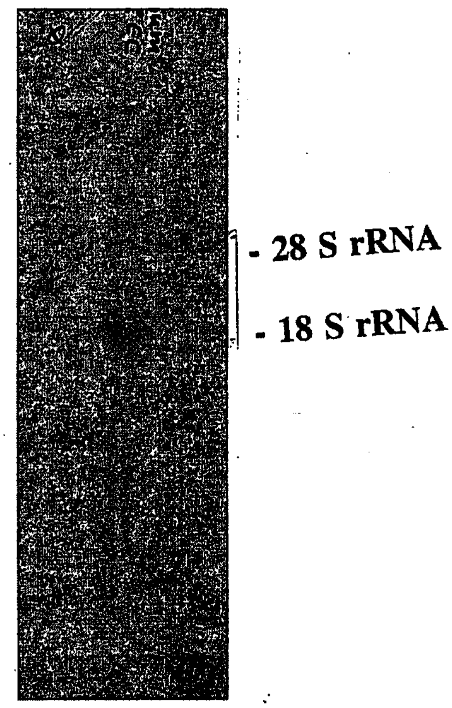


Fig. 5c

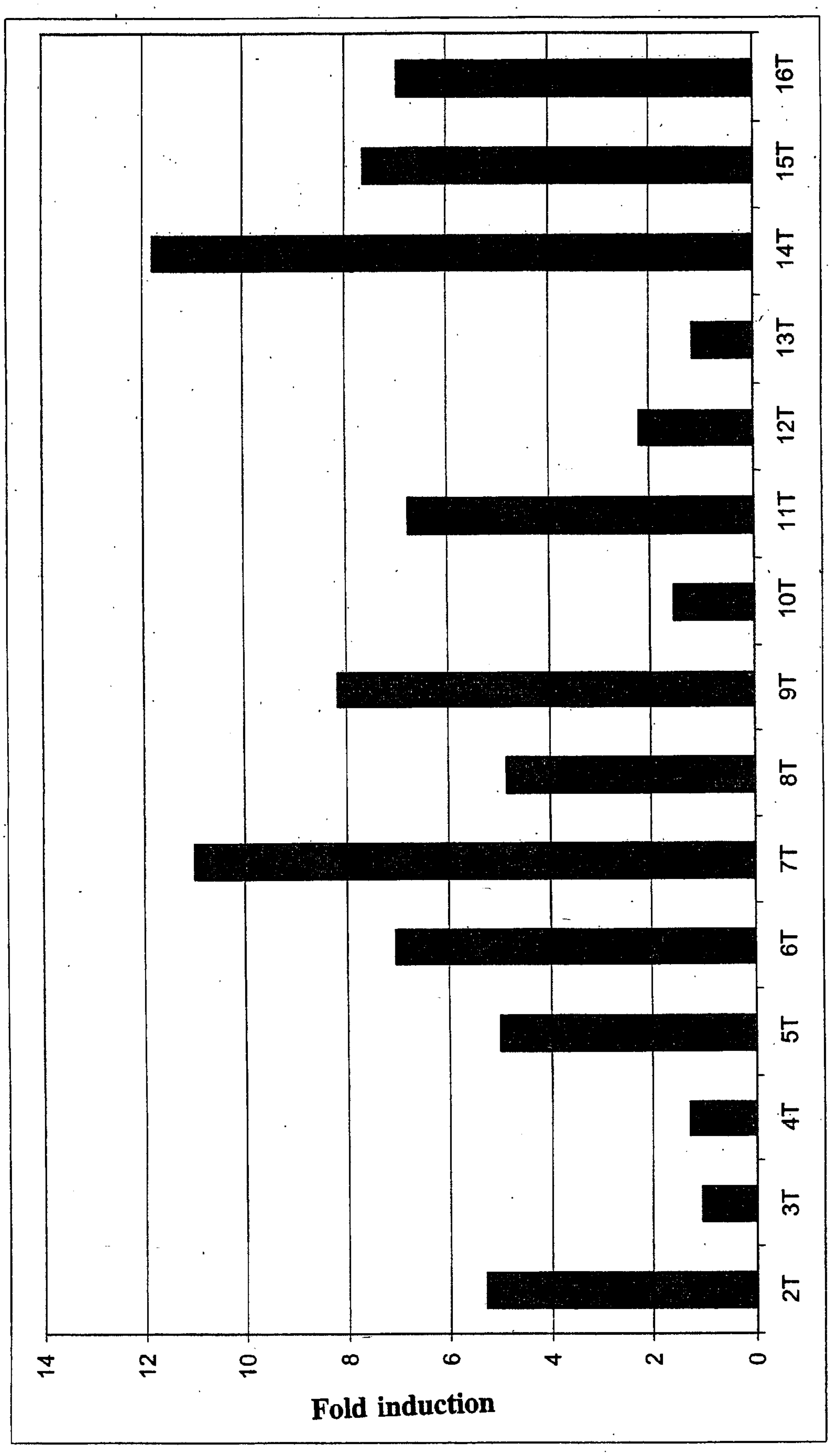
16T
15T
14T
13T
12T
11T
10T
9T
8T
7T
6T
5T
4T
3T
2T
14
12
10
8
6
4
2
0
Fold induction

Fig. 5d

3D-model of mTAP-70

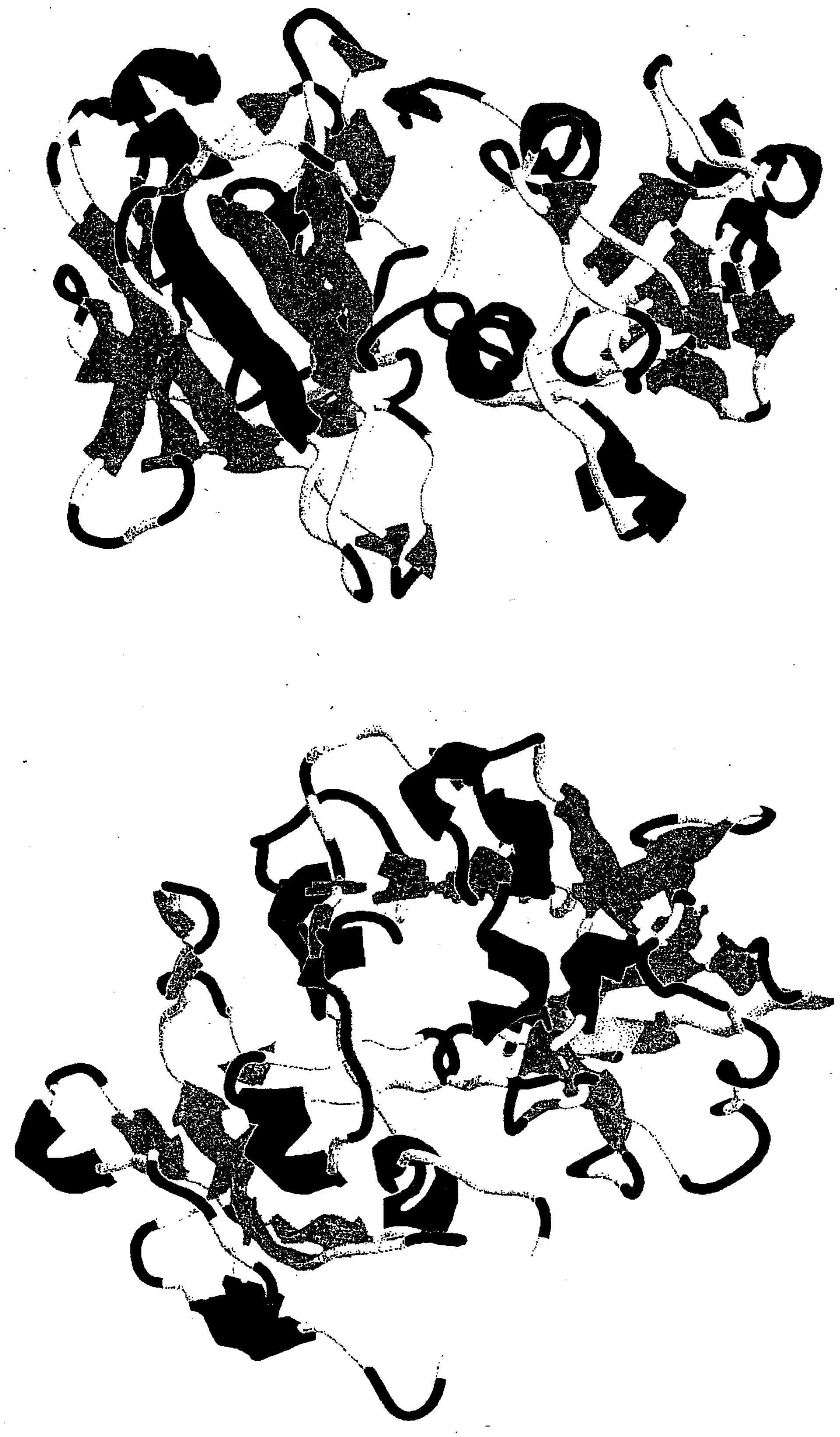

Fig. 6

European Patent Office

# PARTIAL EUROPEAN SEARCH REPORT

which under Rule 45 of the European Patent Convention shall be considered, for the purposes of subsequent proceedings, as the European search report

Application Number EP 02 00 6086

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DATABASE NCBI 'Online!<br>31 January 2002 (2002-01-31)<br>Database accession no. AC079811<br>XP002211968<br>nucleotides 172635-174330<br>--- | 1-20,31 | C12N15/12<br>C12N15/57<br>C12N15/62<br>C12N5/10<br>C12N1/19<br>C12N1/21<br>C12N9/64<br>C07K14/47<br>G01N33/50<br>G01N33/574<br>C12Q1/68<br>A61K38/17<br>A61K38/48<br>A61K48/00<br>A61K31/7088<br>A61P35/00 |
| X | DATABASE NCBI 'Online!<br>31 October 2001 (2001-10-31)<br>Database accession no. AK055994<br>XP002211969<br>--- | 1-20,31 | |
| X | DATABASE EMBL 'Online!<br>31 October 2001 (2001-10-31)<br>Database accession no. AK057813<br>XP002211970<br>---<br>-/-- | 1-20,31 | |

TECHNICAL FIELDS SEARCHED (Int.Cl.7)

C07K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 9 September 2002 | Barnas, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

## INCOMPLETE SEARCH SHEET C

Application Number
EP 02 00 6086

Claim(s) searched incompletely:
12

Claim(s) not searched:
23

Reason for the limitation of the search:

Present claims 12 and 23 relate to an oligopeptide and to an activator or inhibitor defined by reference to a desirable characteristic or property, namely binding to the polypeptide of claim 6 or 8, activating or inhibiting said polypeptide and activating or inhibiting the expression of said polypeptide.

The claims cover all oligopeptides and compounds having this characteristic or property. In the present case, the claims so lack support, and the application so lacks disclosure, that a meaningful search over the whole of the claimed scope is impossible. Independent of the above reasoning, the claims also lack clarity (Article 84 EPC). An attempt is made to define the oligopeptide and the compound by reference to a result to be achieved. Again, this lack of clarity in the present case is such as to render a meaningful search over the whole of the claimed scope impossible. Consequently, the search has been carried out for those parts of the claims which appear to be clear, supported and disclosed, namely those parts described in claims 12 a-c.

European Patent Office

# PARTIAL EUROPEAN SEARCH REPORT

Application Number
EP 02 00 6086

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| X | RIKEN GENOME EXPLORATION RESEARCH GROUP PHASE II TEAM AND FANTOM CONSORTIUM: "Functional annotation of a full-length mouse cDNA collection." NATURE (LONDON), vol. 409, no. 6821, 2001, pages 685-690, XP001009930 ISSN: 0028-0836 * the whole document * --- | 1-20,31 | |
| A | FOEKENS J A ET AL: "Cathepsin-D in primary breast cancer: Prognostic evaluation involving 2810 patients." BRITISH JOURNAL OF CANCER, vol. 79, no. 2, January 1999 (1999-01), pages 300-307, XP002211966 ISSN: 0007-0920 abstract * page 302, right-hand column, paragraph 2 - page 305, left-hand column, paragraph 3 * --- | 21-34 | TECHNICAL FIELDS SEARCHED (Int.Cl.7) |
| A | TRUAN N ET AL: "Expression and clinical significance of pepsinogen C in resectable pancreatic cancer." INTERNATIONAL JOURNAL OF BIOLOGICAL MARKERS, vol. 16, no. 1, January 2001 (2001-01), pages 31-36, XP002211967 ISSN: 0393-6155 abstract * page 33, left-hand column, paragraph 4 - page 34, left-hand column, paragraph 2 * ----- | 21-34 | |

EPO FORM 1503 03.82 (P04C10)